(19) Europäisches Patentamt
European Patent Office
Office européen des brevets

(11) **EP 3 983 052 B1**

(12) **EUROPEAN PATENT SPECIFICATION**

(45) Date of publication and mention
of the grant of the patent:
**20.11.2024 Bulletin 2024/47**

(21) Application number: **20834152.9**

(22) Date of filing: **01.07.2020**

(51) International Patent Classification (IPC):
**C08K 3/013** (2018.01)    **C09C 1/02** (2006.01)
**C09C 1/30** (2006.01)    **C09C 1/40** (2006.01)
**C09D 7/62** (2018.01)    **C09C 1/42** (2006.01)
**C09C 3/00** (2006.01)    **C09C 3/08** (2006.01)
**C09C 3/10** (2006.01)    **C09C 3/12** (2006.01)

(52) Cooperative Patent Classification (CPC):
**A61N 1/375; A61B 5/0059; A61B 5/4836;**
**A61N 1/0553; A61N 1/36062; A61N 1/36071;**
A61B 5/061; A61N 1/3754

(86) International application number:
**PCT/US2020/070224**

(87) International publication number:
**WO 2021/003496 (07.01.2021 Gazette 2021/01)**

(54) **IMPROVED SURGICAL ELECTRODE AND LEAD FOR USE WITH IMPLANTED PULSE GENERATOR**

VERBESSERTE CHIRURGISCHE ELEKTRODE UND LEITUNG ZUR VERWENDUNG MIT EINEM IMPLANTIERTEN IMPULSGENERATOR

ÉLECTRODE CHIRURGICALE AMÉLIORÉE ET SONDE DESTINÉES À ÊTRE UTILISÉES AVEC UN GÉNÉRATEUR D'IMPULSIONS IMPLANTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2019 US 201962869372 P**
**01.07.2019 US 201962869397 P**
**01.07.2019 US 201962869377 P**

(43) Date of publication of application:
**20.04.2022 Bulletin 2022/16**

(73) Proprietor: **Wavegate Corporation**
**Lake Charles, LA 70605 (US)**

(72) Inventor: **WOLF II, Erich, W.**
**Lake Charles, LA 70605 (US)**

(74) Representative: **Forresters IP LLP**
**Skygarden**
**Erika-Mann-Straße 11**
**80636 München (DE)**

(56) References cited:
**US-A- 4 715 700**       **US-A1- 2011 046 700**
**US-A1- 2013 030 352**   **US-A1- 2013 317 572**
**US-A1- 2014 074 182**   **US-A1- 2014 340 741**
**US-A1- 2018 326 219**   **US-A1- 2018 326 219**
**US-A1- 2021 001 115**

**Description**

**FIELD OF THE INVENTION**

The present invention is set out in the appended claims and pertains to a surgical lead. The present description generally relates to an improved implantable pulse generator (IPG) and header combination for using optical reflectometry in spinal cord stimulation (SCS).

**BACKGROUND OF THE INVENTION**

Chronic pain may arise from a variety of conditions, most notably from nerve injury as in the case of neuropathic pain, or from chronic stimulation of mechanical nociceptors such as with spinal pain. Functional ability may be severely impacted by pain, which often is refractory to pharmacological and surgical treatment. In such cases, spinal cord stimulation ("SCS") can be an effective treatment for pain by modulating physiological transmission of pain signals from the periphery to the brain. This may be achieved by applying electrical impulses to the spinal cord via an electrode array implanted adjacent the spinal canal.

Spinal cord stimulator (SCS) system electrode leads may be classified as either "percutaneous leads" or "surgical leads". Percutaneous lead arrays contain multiple cylindrical electrode contacts which are arranged colinear along a thin cylindrical cable which is introduced into the body via a needle. In contradistinction, surgical leads are generally comprised of an array of electrode contacts which protrude on one side from a thin lead body composed of a flexible substrate which is directly placed in the dorsal epidural space via a surgical laminotomy.

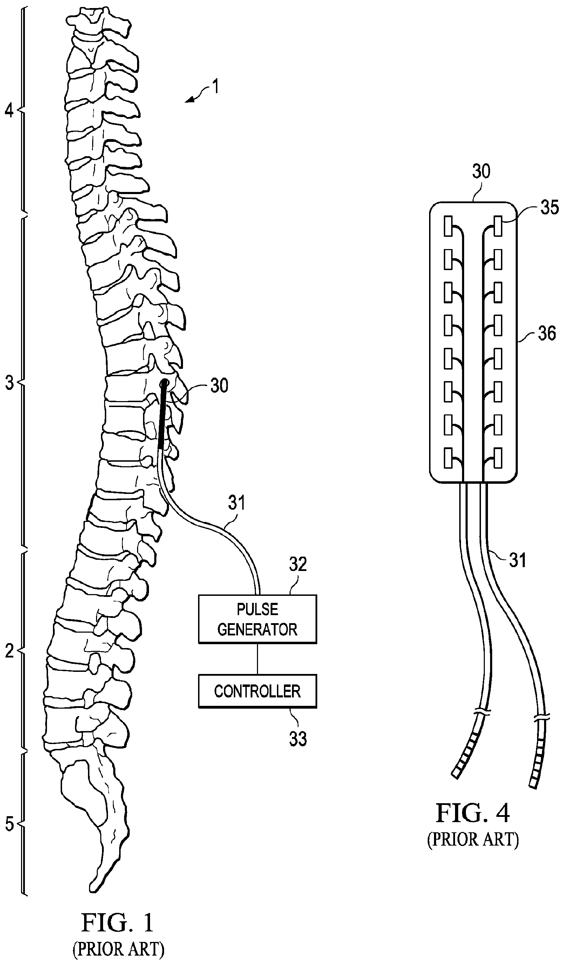

In Figure 1, spinal column **1** is shown to have a number of vertebrae, categorized into four sections or types: lumbar vertebrae **2**, thoracic vertebrae **3**, cervical vertebrae **4** and sacral vertebrae **5**. Cervical vertebrae **4** include the 1st cervical vertebra (C1) through the 7th cervical vertebra (C7). Just below the 7th cervical vertebra is the first of twelve thoracic vertebrae **3** including the 1st thoracic vertebra (T1) through the 12th thoracic vertebra (T12). Just below the 12th thoracic vertebrae **3,** are five lumbar vertebrae **2** including the 1st lumbar vertebra (L1) through the 5th lumbar vertebra (L5), the 5th lumbar vertebra being attached to sacral vertebrae **5** (S1 to S5), sacral vertebrae **5** being naturally fused together in the adult.

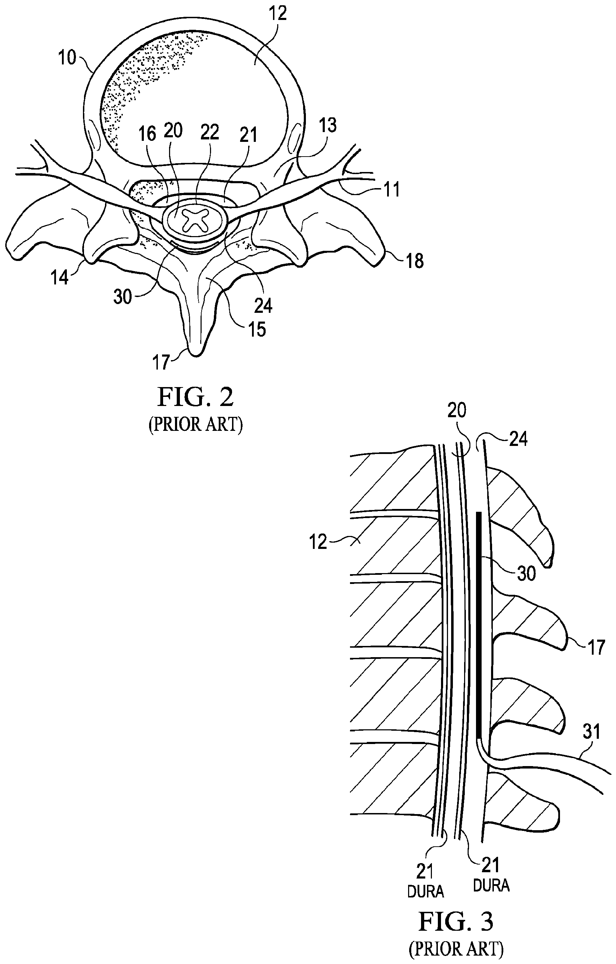

In Figure 2, representative vertebra **10,** a thoracic vertebra, is shown to have a number of notable features which are in general shared with lumbar vertebrae **2** and cervical vertebrae **4**. The thick oval segment of bone forming the anterior aspect of vertebra **10** is vertebral body **12**. Vertebral body **12** is attached to bony vertebral arch **13** through which spinal nerves **11** run. Vertebral arch **13,** forming the posterior of vertebra **10,** is comprised of two pedicles **14,** which are short stout processes that extend from the sides of vertebral body **12** and bilateral laminae **15**. The broad flat plates that project from pedicles **14** join in a triangle to form a hollow archway, spinal canal **16**. Spinous process **17** protrudes from the junction of bilateral laminae **15**. Transverse processes **18** project from the junction of pedicles **14** and bilateral laminae **15**. The structures of the vertebral arch protect spinal cord **20** and spinal nerves **11** that run through the spinal canal.

Surrounding spinal cord **20** is dura **21** that contains cerebrospinal fluid (CSF) **22.** Epidural space **24** is the space within the spinal canal lying outside the dura.

Referring to Figures 1, 2 and 3, the placement of an electrode array for spinal cord stimulation according to the prior art is shown. Electrode array **30** is positioned in epidural space **24** between dura **21** and the walls of spinal canal **16** towards the dorsal aspect of the spinal canal nearest bilateral laminae **15** and spinous process **17.**

Figure 4 shows a prior art surgical electrode array **30** including electrode contacts **35** sealed into elastomeric housing **36.** Electrode array **30** has electrode leads **31** which are connected to electrical pulse generator **32** and controller **33.** Each electrode contact has a separate electrical conductor in electrode leads 31 so that the current to each contact may be independently controlled.

Spinal cord stimulators often include an implantable pulse generator (IPG) **32** which monitors and delivers the electrical stimulation to the spinal cord through the electrode array **31.** The IPG is typically contained in a titanium canister which is implanted subcutaneously near the upper buttocks or flank and draws power from a battery. The electrode array is connected to the IPG using subcutaneous leads.

The subcutaneous leads interface with electrode contacts located in the header of an IPG. Typically, the leads are secured in the IPG with an anchor screw.

The IPG delivers pulses of electrical current to the electrode array, which travel through the electrodes to targeted neurons within the ascending tracts of the spinal cord. The resulting electric field disrupts the perception of pain. Controlling the amplitude of the stimulating electrical field is paramount to success of spinal cord stimulation. Applying inadequate current will fail to depolarize the targeted neurons, rendering the treatment ineffective. Conversely, application of excess current will depolarize the targeted neurons, but also stimulate additional cell populations which renders the perception of a noxious stimulation.

Establishing a consistent, therapeutic, and non-noxious level of stimulation is predicated upon establishing an ideal current density within the spinal cord's targeted neurons. Fundamentally, this should be a simple matter of establishing an optimal electrode current given the local bulk conductivity of the surrounding tissues. But in practice, the optimal electrode current changes as a function of patient position and activity due to motion of the spinal cord as the spinal cord floats in cerebrospinal fluid within the spinal canal. Significant changes in distance between the epidural electrode array and the targeted spinal cord neurons have been shown to occur. Consequently, optimal stimulation requires dynamic adjustment of the electrode stimulating current as a function of distance between the electrode array and the spinal cord.

Dynamic modulation of spinal cord stimulator electrode current as a function of distance between the electrode array and the spinal cord thus has several benefits. Excess stimulation current can be avoided, thus reducing the prospects of noxious stimulation and potentially reducing device power consumption. Inadequate stimulation current can also be avoided, thus eliminating periods of compromised therapeutic efficacy.

Dynamic modulation of electrode current can be controlled through the use of optical reflectometry to determine the thickness of the dorsal cerebrospinal fluid (dCSF) column between the spinal cord and the electrode array. An optical signal is transmitted into the surrounding tissue and collected by a sensor to calculate the approximate distance between the electrode and the spinal cord. The stimulus magnitude is modified accordingly to provide the optimal current for pain relief. An example of this technology is shown in U.S. Patent No. 10,035,019 to Wolf II.

One challenge to subcutaneous IPG implants is the long-term survival of the IPG in the harsh *in vivo* environment. Functional and mechanical degradation may occur with the ingress of body fluids. Proteins common in the blood and interstitial fluid are known to bind to metallic ions, leading to corrosion. Some materials can trigger an immune response and potentially a change in the local pH balance of the implantation site. Specialized polymers and epoxies can avoid some of these problems, but often exhibit unacceptably high levels of cytotoxicity. Consequently, it is imperative to maintain the IPG internal components in a hermetically sealed environment and that the external IPG components be biocompatible.

Similarly, another challenge to subcutaneous IPG implants is the tendency for the surrounding tissue to degrade around the IPG due to increased pressure the IPG edges place on the tissue. Erosion of the device through the skin can occur, typically at the corners of the device where there is a focal concentration of pressure, and requires revision surgery to replace the device.

Another challenge to implementation of optical reflectometry for adaptive spinal cord stimulation is that leads coupled imprecisely to the IPG header are susceptible to movement which interferes with the stability of the optical signal. Unstable optical signals result in undesirable signal-to-noise ratio which results in errors in delivered current and imprecise stimulation.

Yet another challenge to subcutaneous IPG implants is the extended recharge times. IPGs including a rechargeable battery must be periodically recharged. Electromagnetic induction has evolved as the most widely used technology for recharging IPG batteries. However, during recharging, eddy currents are produced in the IPG casing causing temperature increase. To maintain an acceptable temperature, charging duty cycles are typically shorter than ideal, thereby increasing the time required for recharging.

The prior art has attempted to address these challenges in a number of ways.

For example, U.S. Patent No. 6,011,993 to Tziviskos, et al. describes a method of making a strong ceramic case that can house electronics with a good hermetic seal for implantation into the body. However, *Tziviskos* does not describe how to effectively connect or secure electrical leads or optical fibers.

As another example, U.S. Patent No. 6,324,428 to Weinberg, et al. describes a medical implant that contains the internal electronics in a preferred configuration that minimizes the volume of the implant, making it easier to implant. However, *Weinberg* does not describe any design feature that reduces device erosion, nor does it disclose how to couple electrical leads or optical fibers to the implant.

Similarly, U.S. Patent No. 7,742,817 to Malinowski, et al. describes an IPG with connectors for electrical leads and an epoxy coating for biocompatibility. However, *Malinowski* does not disclose the use of optics in the design to achieve proper pulse strength.

U.S. Publication No. 2014/0074182 to Wolf, discloses a positionally sensitive spinal cord stimulation apparatus using near-infrared (NIR) reflectometry for automatic adjustments of spinal cord stimulation. The system comprises an electrode assembly with an optical fiber sensor for sensing spinal cord position. The optical fiber sensor, comprising a set of optical elements for emitting light from a set of IR emitters and for collecting reflected light into a set of IR photodetectors, determines a set of measured optical intensities. US2018/326219 also discloses a known spinal cord stimulation system.

Deficiencies exist in the prior art related to the accuracy of lead coupling when using optical reflectometry for spinal cord stimulation. Thus, there is a need in the art for an improved IPG case, connectors, leads and electrodes which provide a stable optical signal while optimizing the longevity of the IPG.

## BRIEF DESCRIPTION OF THE DRAWINGS

In the detailed description of the preferred embodiments presented below, reference is made to the accompanying drawings.

Figure 1 is a side view of the human spine showing the approximate position of an electrode array for spinal cord stimulation.

Figure 2 shows an axial view of a thoracic vertebra indicating the position of the spinal cord and an electrode array for spinal cord stimulation.

Figure 3 shows a sagittal cross-sectional view of the human spine showing the approximate position of an electrode array for spinal cord stimulation.

Figure 4 shows a prior art surgical electrode array and lead connector for spinal cord stimulation.

Figure 5 shows a schematic of an IPG charging and communication system of a preferred embodiment.

Figure 6A is an isometric view of a preferred IPG device.

Figure 6B is a cross-sectional top view of a preferred IPG shape demonstrating a super ellipse curve.

Figure 6C is a cross-sectional front view of a preferred IPG shape demonstrating a super ellipse curve.

Figure 6D is cross-sectional side view of a preferred IPG shape demonstrating a super ellipse curve.

Figure 6E is an isometric view of a preferred IPG shape demonstrating a super ellipse curve.

Figure 6F is an exploded isometric view of a preferred IPG device.

Figure 7A is a side view of a header for a preferred IPG device.

Figure 7B is a cross-sectional top view of a header for a preferred IPG device.

Figure 7C is a detail view a preferred header for an improved IPG device.

Figure 7D is a top view of a preferred header bay for an improved IPG device.

Figure 7E is a rear view of a header for an improved IPG device.

Figure 8 is a cross-sectional view of a preferred IPG body.

Figure 9A is a plan view of an optical window for an improved IPG device.

Figure 9B is a cross-sectional side view of an optical window for an improved IPG device.

Figure 10A is a plan view of an optical window for an improved IPG device.

Figure 10B is a cross-sectional side view of an optical window for an improved IPG device.

Figure 11A is a plan view of an optical window for an improved IPG device.

Figure 11B is a cross-sectional side view of an optical window for an improved IPG device.

Figure 11C is an isometric view of an optical window for an improved IPG device.

Figure 11D is an isometric view of an optical window for an improved IPG device.

Figure 12A is a front view of a preferred daughterboard for an improved IPG device.

Figure 12B is a rear view of a preferred daughterboard for an improved IPG device.

Figure 12C is an isometric view of a preferred daughterboard for an improved IPG device.

Figure 12D is a schematic of an optical signal for an improved IPG device.

Figure 12E is a graphical depiction of the advantages of a lead configuration.

Figure 12F is a method diagram for calculating stimulation.

Figure 12G is a front view of a daughterboard for an improved IPG device.

Figure 12H is a rear view of a daughterboard for an improved IPG device.

Figure 12I is an isometric view of a daughterboard for an improved IPG device.

Figure 12J is a schematic of an optical signal for an improved IPG device.

Figure 13A is a side view of a preferred embodiment of subcutaneous leads.

Figure 13B is a cross-sectional view of a preferred embodiment of subcutaneous leads.

Figure 13C is a cross-sectional view of an alternative embodiment of subcutaneous leads.

Figure 13D is an exploded side view of an optical fiber and ferrule configuration.

Figure 13E is a side view of a preferred embodiment of an optical fiber and ferrule assembly.

Figure 13F is an exploded side view of an optical fiber and collet assembly.

Figure 13G is a side view of a preferred embodiment of an optical fiber and collet assembly.

Figure 13H is an exploded side view of a lead assembly.

Figure 13I is a plan view of an optical fiber threading assembly.

Figure 13J is a plan view of an optical fiber threading assembly.

Figure 13K is an exploded perspective view of an optical fiber threading assembly.

Figure 14A is a plan view of a preferred surgical lead.

Figure 14B is a cross-sectional view of a preferred surgical lead.

Figure 14C is a cross-sectional view of a preferred surgical lead.

Figure 15A is a plan view of a preferred surgical lead.

Figure 15B is a cross-sectional view of a preferred surgical lead.

Figure 16A is a plan view of a surgical lead.

Figure 16B is a cross-sectional view of a surgical lead.

Figure 16C is an isometric view of a parabolic reflector for a surgical lead.

Figure 17A is a plan view of a surgical lead.

Figure 17B is a cross-sectional view of a surgical lead.

Figure 18 is flowchart of the steps of a preferred method of placement of a surgical lead.

Figure 19 is flowchart of the steps of a preferred method of placement of a percutaneous lead.

Figure 20 is flowchart of a method of the steps of a preferred method of securing an optical fiber in a stylet channel of a lead.

## DETAILED DESCRIPTION

According to the present invention, there is provided a surgical lead as recited by Claim 1. Further, preferable features are presented in the dependent claims.

In the description that follows, like parts are marked throughout the specification and figures with the same numerals, respectively. The figures are not necessarily drawn to scale and may be shown in exaggerated or generalized form in the interest of clarity and conciseness.

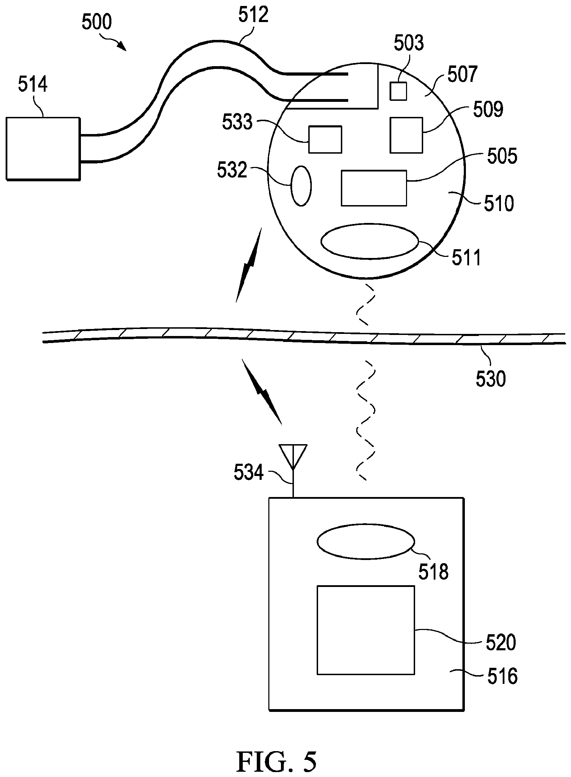

Referring then to Figure 5, IPG charging and communication system **500** comprises an IPG device **510** implanted subcutaneously beneath skin surface **530**.

IPG device **510** comprises an external non-metallic case **507** which facilitates transmission of charging and communication signals, with external system manager **516**, as will be further described.

IPG device **510** further comprises main processor **505**, operatively connected to signal processor **509**. Main processor **505** is further operatively connected to secondary coil **511** and RF antenna **532**, as will be further described.

Signal processor **509** is operatively connected to optoelectrical devices **503**, as will be further described.

Optoelectrical devices **503** are positioned to send and receive light into and out of, respectively, leads **512** of surgical lead **514**, as will be further described.

Main processor **505** is further operatively connected to battery **533**, secondary coil **511** and RF antenna **532**. In use, main processor **505** mitigates charging battery **533** from current induced in secondary coil **511**, by primary coil **518**, as will be further described. Main processor **505** further receives signals from RF antenna **532**, for use in communicating data regarding operation of the IPG device, as will be further described.

The system further comprises external system manager **516**. External system manager **516** includes external processor **520**, operatively connected to primary coil **518** and RF antenna **534**.

In use, external processor **520** includes a set of instructions which control a charging signal sent to primary coil **518**. In use, primary coil **518** is placed physically near secondary coil **511** and activated. The activation of the primary coil induces a current in the secondary coil which is routed to the battery by the main processor for charging the battery. The activation of the primary coil and the inductive charging of the battery can be continuous since there are no eddy currents created in the non-metallic case. A continuous charging duty cycle for an IPG is a significant improvement over the prior art which reduces IPG charging time.

RF antenna **534** is used to send and receive signals to RF antenna **532** to receive information and control operation of IPG device **510**, as will be further described.

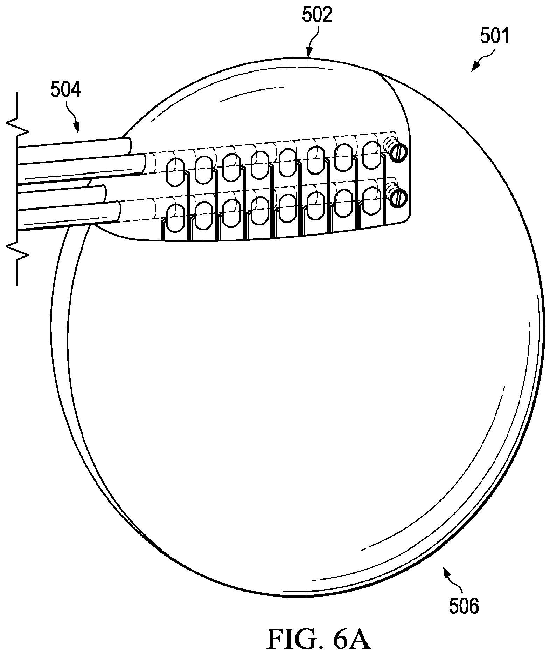

Referring then to Figure 6A, IPG device **501** comprises IPG body **506** and header **502**. Leads **504** are removably secured in the header, as will be further described.

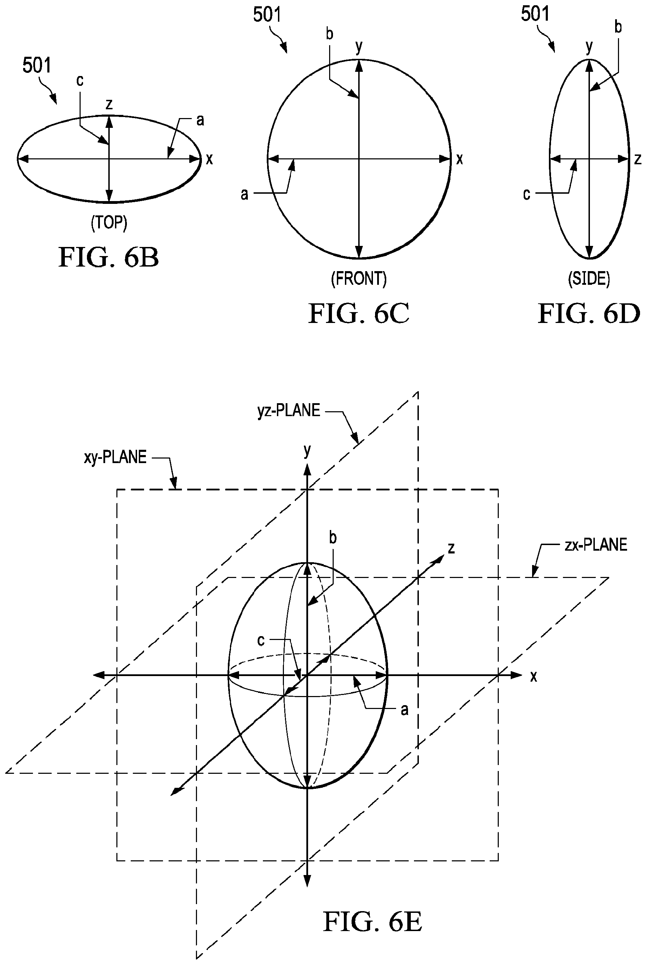

Referring then to Figures 6B, 6C, 6D and 6E, the preferred shape for IPG device **501** will be described. In general, the preferred shape of the IPG case is defined by two (2) unique super ellipse equations, one for each of the side and top perspectives. The case is symmetrical about each principal axis. The external shape of the IPG case is important because a near Gaussian distribution of curvatures over the surface greatly reduces the risk of erosion of the case through the skin after implantation of the IPG device, thereby increasing the survivability of the surgical implant. The preferred super ellipse equations which define the shape of the case are preferably Lamé curve equations.

The device three-dimensional shape is a volume of revolution having principle axes *x, y* and *z*. The volume of revolution is symmetrical about each principle axis. Referring to Figure 6C, from the front, in the *x y* plane, the volume of revolution is preferably a circle, defined by the equation:

$$\left| \frac{a}{2} \right|^2 + \left| \frac{b}{2} \right|^2 = r^2 \qquad\qquad \text{Eq. 1}$$

where:

5

$a$ = width along the $x$ axis;
$b$ = height along the $y$ axis;
$r$ = radius.

Typical values for $a$ and $b$ are about 50 mm. A typical value for $r$ is about 25 mm.

Referring to Figure 6D, from the side, in the $y\,z$ plane the volume of revolution is preferably a super-ellipse defined by the equation:

$$\left|\frac{z}{c}\right|^{n} + \left|\frac{y}{b}\right|^{n} = 1 \qquad \text{Eq. 2}$$

where:

$b$ = height along the $y$ axis;
$c$ = depth along the $z$ axis;
$n$ is between about 1.5 and about 5, and is preferably about 2.

A typical value for $b$ is about 50 mm. A typical value for $c$ is about 12 mm.

In one preferred embodiment, the super ellipse in the $y\,z$ plane is rotated about the $z$ axis to obtain the volume of revolution.

Referring to Figure 6B, from a top, in the $\tau z$ plane, the volume of revolution is preferably a super-ellipse defined by the equation.

$$\left|\frac{x}{a}\right|^{n} + \left|\frac{z}{c}\right|^{n} = 1 \qquad \text{Eq. 3}$$

where:

$a$ = width along the $x$ axis;
$c$ = depth along the $z$ axis;

A typical value for $a$, is about 50 mm. A typical value for $c$, is about 12 mm.

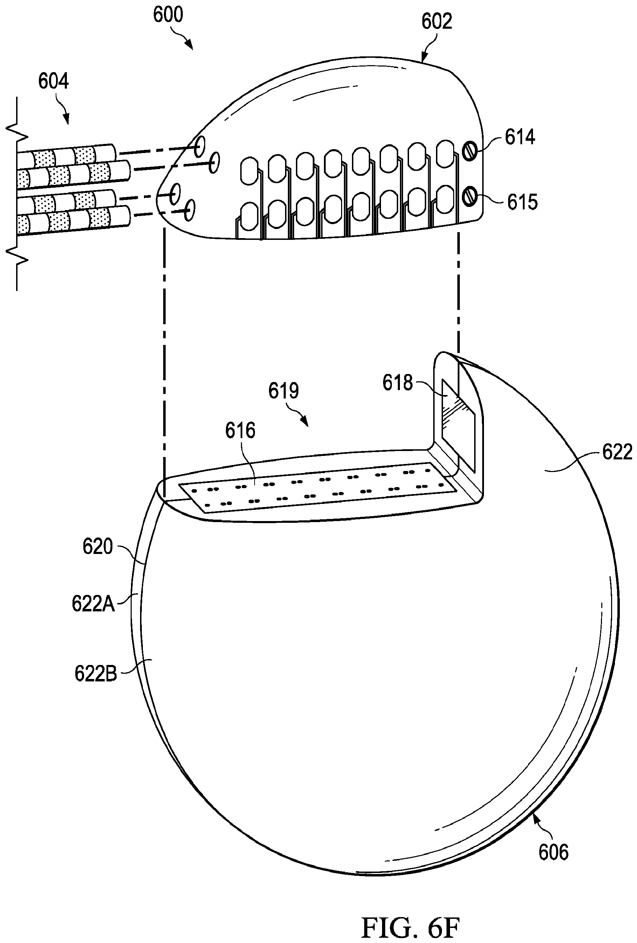

Referring then to Figure 6F, an exploded view of improved IPG device **600** will be described.

IPG device **600** is comprised of header **602** and IPG body **606**. IPG body **606** is further comprised of IPG casing **622**, optical window **618** and electrical feedthrough plate **616**. IPG casing **622** is formed by two opposing shell halves, **622a** and **622b**, hermetically sealed at junction **620**. In a preferred embodiment, IPG casing **802** is a ceramic material, such as alumina, sapphire or zirconia. In another embodiment, the IPG casing may be formed of a molded amorphous glass, such as Pyrex®. In alternative embodiments, the IPG casing may be comprised of titanium or an alloy. In a preferred embodiment, ceramic brazing with induced welding is applied at the junction of the casing halves. Other processes may be used to join the halves.

The header is fixed in header bay **619** by a suitable medical grade permanent adhesive, as will be further described.

Optical window **618** is preferably a crystal insert in a wall of the header bay that is hermetically sealed in the IPG casing, as will be further described. Alternatively, in embodiments where the IPG casing is formed of an optically transparent material, optical window **618** may take the form of a pair of polished surfaces integrally formed in the header bay wall, of the IPG casing, adjacent the header body.

Leads **604** are removably coupled with header **602** and secured in place using anchor screws **614** or **615**, as will be further described.

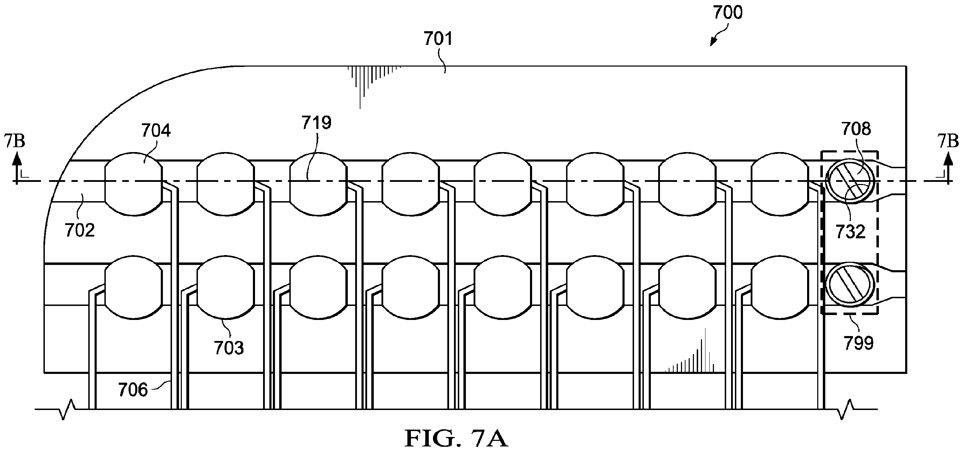

Referring then to Figure 7A, header **700** is comprised of header body **701**. The header body is preferably formed of a cast rigid non-metallic material of sufficient strength to support radial forces from the anchor screws, such as methyl PMMA or polyester reinforced with fiberglass or graphite fibers. The header body includes a plurality of generally latitudinal and parallel lead channels, such as lead channel **702**. In a preferred embodiment, the header body includes four lead channels. Alternatively, it may have two lead channels. Each lead channel, such as lead channel **702** is generally cylindrical and includes a lead channel axis, such as axis **719**, which forms an optical axis for the lead, as which will be further described.

Each lead channel includes eight annular connector bays such as connector bay **703**, formed inline on the interior of each channel. Connector bays **703** are equally spaced along the channel axis of each lead channel. Each connector bay houses a canted coil connector spring, such as canted coil spring **704**. Each canted coil connector spring is a helical metallic coil which forms a toroid and which is spring loaded to exert an internally directed radial bias against a metallic lead connector, as will be further described. Preferably, the canted coil springs are platinum alloy to assure failsafe electrical and mechanical contact with the lead contacts. In a preferred embodiment, the canted coil springs are Bal Conn® for Neuromodulation available from Bal-Seal Engineering of Foothill Ranch, California. Each of the canted coil springs is connected to one connector pin, such as connector pin **706,** located at the base of the header.

Header body **701** includes a set of horizontal threaded holes, perpendicular to the lead channels, such as threaded hole **732,** adjacent the IPG casing, extending from the exterior of the header body to the lead channel. An anchor screw, such as anchor screw **708,** is located in each threaded hole.

In a preferred embodiment, the threaded holes are tapped or cast directly into the header body or alternatively cast into the IPG casing. This configuration is important because it eliminates the need for a separate anchoring block in the header and conserves space by incorporating these components into the IPG casing. Furthermore, placement of the anchoring screw nearest the proximal end of the lead channel provides a secure mechanical connection of the lead closest to the optical components, promoting a stable optical signal.

Optionally, the header body may further comprise an integrally formed anchor block **799**. In this embodiment, the threaded holes and anchor screws are resident in the anchor block adjacent the optical window. The anchor block is preferably a medically inert metal such as titanium molded into the header body.

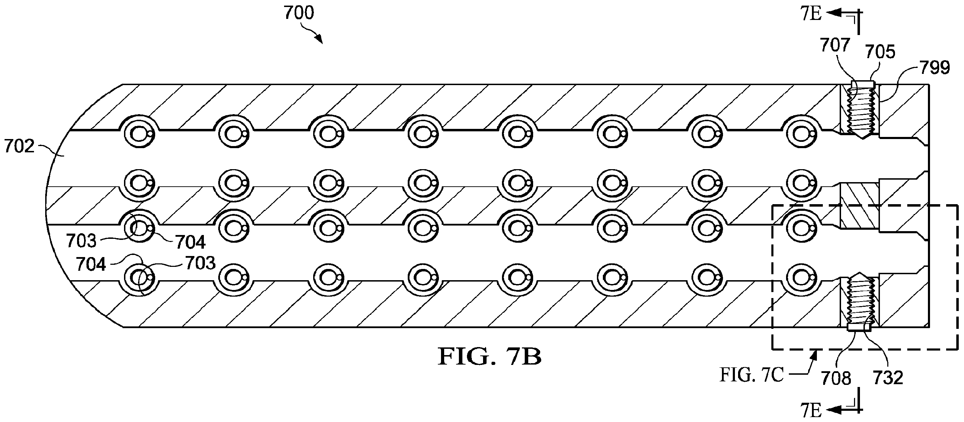

Referring then to Figure 7B, threaded hole **732** houses anchor screw **708.** Diametrically opposed to threaded hole **732** is threaded hole **707.** Threaded hole **707** houses anchor screw **705.**

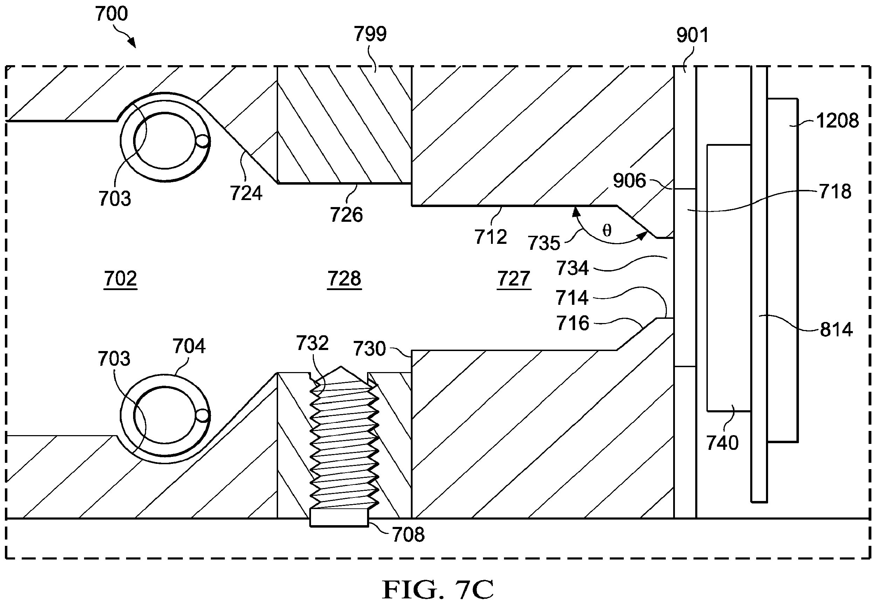

Referring then to Figure 7C, frustoconical centering surface **724** is adjacent to and coaxial with lead channel **702**. Frustoconical centering surface **724** centers the lead on the optical axis of the lead channel as it is inserted into the lead channel. The frustoconical centering surface is adjacent anchor ring chamber **728**. The anchor ring chamber is bounded by cylindrical alignment surface **726** and is coaxial with the frustoconical centering surface. The anchor ring chamber is also bounded by stop surface **730**. Stop surface **730** is an annular ring at the proximal end of the anchor ring chamber. The stop surface is coaxial with the anchor ring chamber. In use, stop surface **730** abuts the proximal end of the lead body and prevents it from being inserted past the desired point in lead channel **702** during assembly. Each of these surfaces is important for accurate positioning of the lead and the optical fiber and promotes efficient and accurate optical signal transfer.

Anchor screw **708** engages the lead anchor ring in the anchor ring chamber when the IPG is assembled. In the case where the threaded holes are formed in the header body, the anchor screw is installed with a torque limited driver to prevent excess force from being placed on the header. In the case where the header body includes an anchor block, the anchor block allows sufficient axial force to be applied by the anchor screw to the anchor ring to hold it securely in place, without fracturing the header body.

The lead channel is further comprised of ferrule chamber **727** bounded by alignment cylinder **712**. The ferrule chamber is coaxial with the lead channel.

Ferrule centering surface **716** is adjacent to and coaxial with alignment cylinder **712** and is designed to hold the ferrule and the optical fiber in optical alignment with the optical axis of the lead channel. Alignment cylinder **712** forms chamfer angle θ, with ferrule centering surface **716**. In a preferred embodiment, chamfer angle θ can range from about 135° to about 150°, $\pm$ 5°. Ferrule centering surface **716** centers and aligns the proximal end of the lead and optical ferrule with buffer gap **734,** optical window **718** and composite optoelectronic device **740**.

Cylindrical buffer surface **714** is adjacent to and coaxial with ferrule centering surface **716**. Cylindrical buffer surface **714** forms buffer gap **734** between the proximal end of the optical ferrule and optical window **718**. The buffer gap prevents application of pressure to the optical window from fluid or tissue build up on the ferrule tip or from irregularities of the optical fiber polished surface at the ferrule tip.

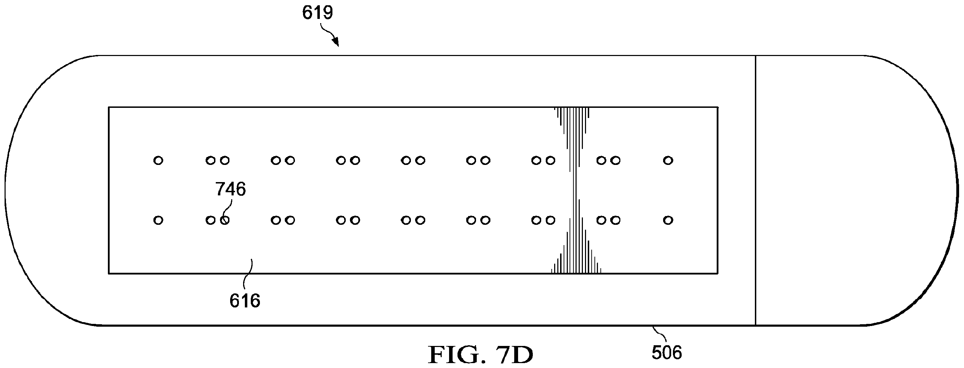

Referring then to Figure 7D, electrical feedthrough plate **616** comprises a flat insulator, preferably a ceramic material, and is fixed at the bottom of header bay **619** by a suitable adhesive, or by ceramic welding. Electrical feedthrough plate **616** is comprised of a plurality of receivers, such as receiver **746**. The receivers are connected to the main circuit board, as will be further described. Connector pins **706** at the base of the header body interface with the receivers.

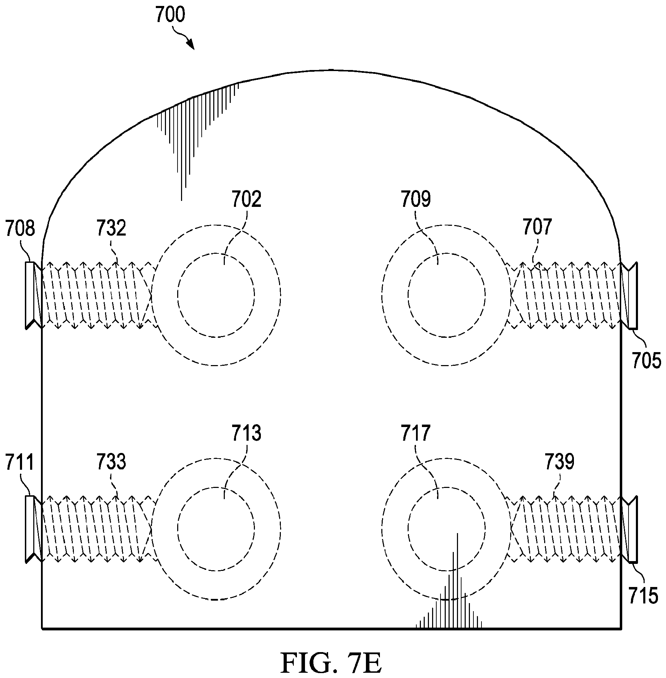

Referring then to Figure 7E, in a preferred embodiment, the header is comprised of four lead channels **702, 709, 713,** and **717.** Each lead channel includes a perpendicularly oriented threaded hole **732, 707, 733** and **739** and anchor screws **708, 705, 711** and **715,** respectively.

In the prior art, there is typically an anchor ring, which is engaged by a set-screw to fix the lead contacts within the header. The anchor ring is typically placed distal to the contacts, requiring a separate anchoring block to engage the lead and set-screw. One advantage of this embodiment is that the anchor ring may be positioned proximal to the lead contacts, nearest the end of the lead. This positioning eliminates the need for a separate anchoring block and reduces the size of the IPG casing if threaded holes **732, 707, 733,** and **739** are integrated into the header body as may be achieved through injection molding of a ceramic or glass. Further, placement of the anchoring ring nearest the proximal tip of the lead provides mechanical fixation of the lead closest to the optical components, promoting a stable optical signal.

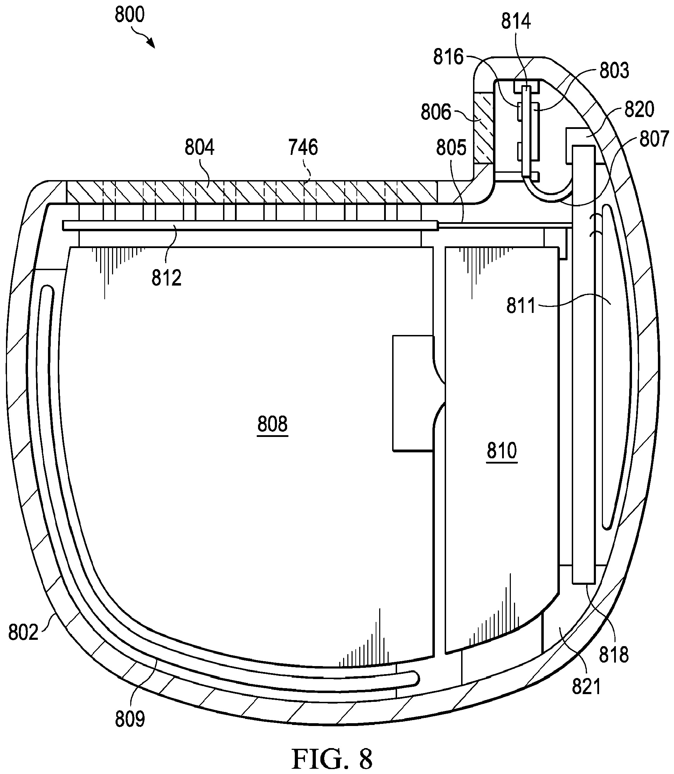

Referring then to Figure 8, IPG body **800** is further comprised of IPG casing **802,** optical window **806,** electrical feedthrough plate **804,** composite optoelectronic device **816,** connector card **812,** main circuit board **818,** battery **808,** and capacitor **810.**

The electrical components are secured in the casing with appropriate insulated plastic standoffs, such as standoffs **820** and **821.**

Electrical feedthrough plate **804** is hermetically sealed to IPG casing **802,** adjacent the header bay. The electrical feedthrough plate is mechanically fixed to connector card **812** and is connected to main circuit board **818** by flexible ribbon cable **805.**

Optical window **806** is hermetically sealed to the IPG casing in a position perpendicular to both the electrical feedthrough plate and the lead channels. In a preferred embodiment, optical window **806** is comprised of synthetic sapphire. Synthetic sapphire provides optimal optical properties for transmitting visible red or infrared light between composite optoelectronic device **816** and optical transmission fibers, as will be further described.

Composite optoelectronic device **816** is positioned adjacent the optical window and held in position parallel to the optical window by the daughterboard. The optoelectronic device **816** is also perpendicular to the optical axis of the lead channels. Daughterboard **814** is further comprised of processor **803,** as will be further described. Daughterboard **814** is held in position by the standoffs and is connected to main circuit board **818** by ribbon cable **807.** The ribbon cable supplies power to the daughterboard and communicates control signals as required.

Main circuit board **818** is positioned in the IPG casing by the standoffs and is operatively connected to the battery, the capacitor, the contacts of the leads and the daughterboard.

Main circuit board **818** receives data input from the daughterboard and generates stimulation pulses which vary in frequency, pulse-width, and amplitude based on signals from the daughterboard. The stimulation pulses are sent to the lead contacts for transmission to the electrodes. The daughterboard generates control signals for the main circuit board by sending light pulses from the light emitters and receiving and interpreting signals from light detectors, as will be further described. The main circuit board is also operatively connected to secondary induction coil **809** and RF antenna **811.**

The main circuit board includes processors and radio signal generators which allow it to communicate signals to exterior receiving devices through RF antenna **811.** In a preferred embodiment, the main processor and the RF antenna are used to communicate a warning signal from the daughterboard if an emitter current reaches a maximum value, as will be further described.

Capacitor **810** is connected to battery **808** and stores energy from the battery to produce the stimulation pulses. In a preferred embodiment, battery **808** is a lithium-ion rechargeable battery. Battery **808** is inductively charged through secondary induction coil **809** positioned around the battery on one internal surface of the IPG casing. The main circuit board controls the recharging duty cycle.

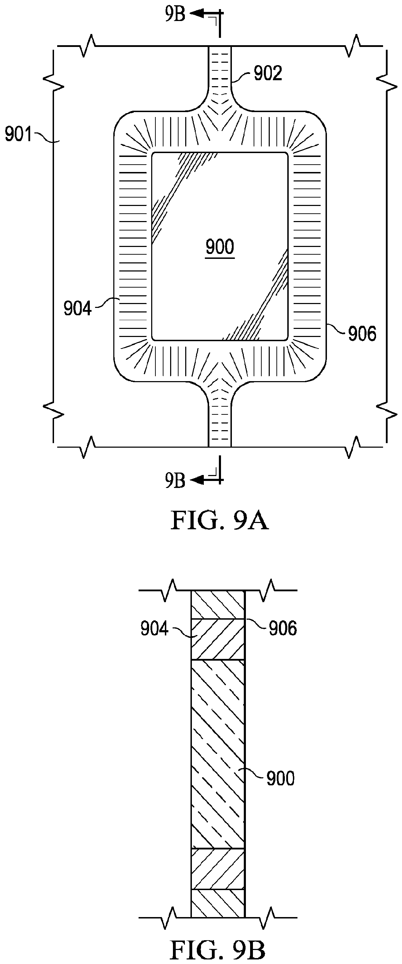

Referring then to Figures 9A and 9B, in a preferred embodiment, optical window **900** is a polished rectangle single crystal alumina (sapphire) or polycrystalline alumina ceramic. It is joined to IPG case **901** in the header bay by ceramic brazing. Niobium is used as a metal to ceramic filler material. In a preferred embodiment the alumina is 94% brazed to Fe-29Ni-10Co internally at approximately 1000° C. Optical window **900** is brazed to IPG case **901** along window braze junction **906** using hermetic braze fillet **904.** In Figure 9B, optical window **900** and IPG case **901** are shown as coplanar, but these may alternatively be stacked or overlaid.

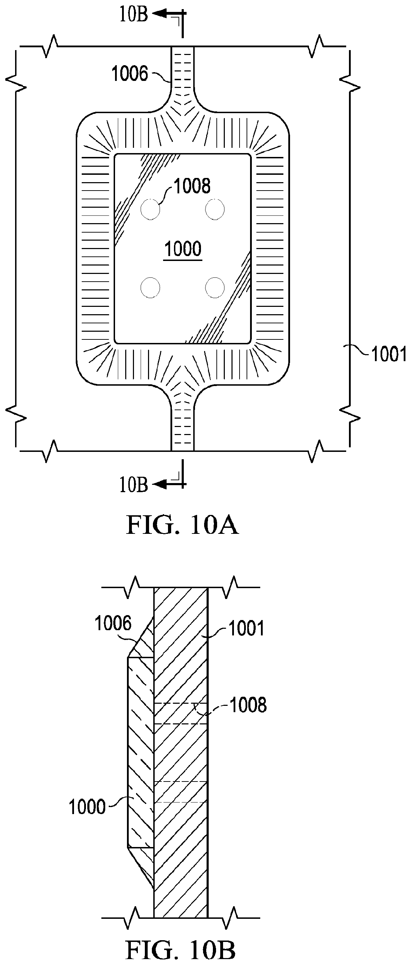

Referring then to Figures 10A and 10B, in another embodiment, optical window **1000** is overlaid on the outside of IPG case **1001,** adjacent the header bay. In this embodiment, IPG case **1001** includes four (4) waveguides **1008.** The waveguides are holes in the header bay wall that allow red or infrared light to be transmitted through the optical window, along the optical axis of each lead channel and into the interior of the IPG casing. Optical window **1000** is hermetically sealed to IPG case using brazing, soldering, epoxy or other suitable means along window junction **1006.**

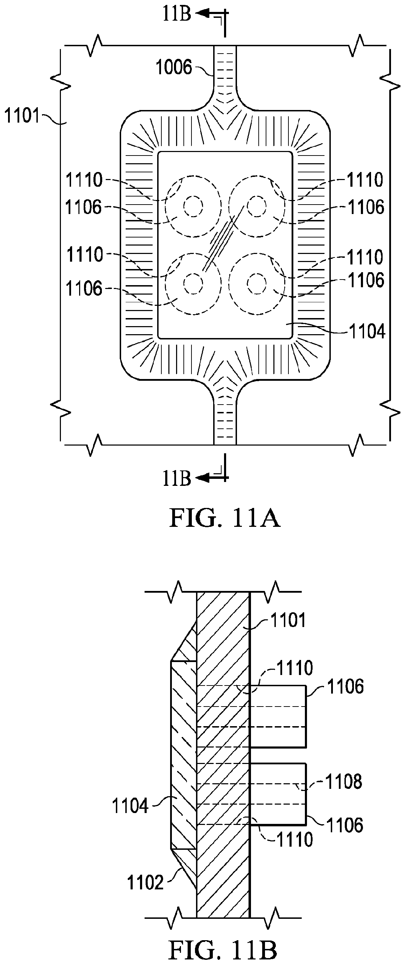

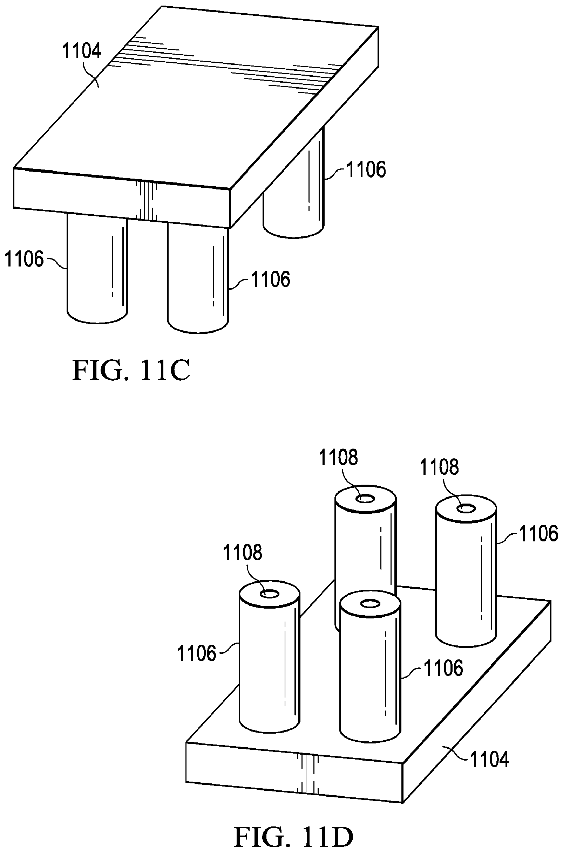

Referring then to Figures 11A, 11B, 11C and 11D, in another preferred embodiment, window plate **1104** comprises a flat sapphire rectangle about 1 mm thick. Four optical wave guides, **1106** are fused to the window plate using ceramic welding. In another embodiment, the window plate and optical waveguides are integrally formed from the same crystal structure. Each optical wave guide includes an internally reflective iris **1108.** The iris is a cylindrical hole which is concentrically aligned with the optical axis of a lead channel. Window plate **1104** is laser welded to IPG casing **1101** along weld joint **1102.**

When assembled, each of the optical wave guides passes through holes **1110** and into the interior of the IPG casing. In a preferred embodiment, each optical wave guide abuts an optoelectronic device on the daughterboard secured in the IPG casing, as previously described. In practice, the iris is important because it prevents light loss between the optical fiber in the lead and the optoelectronic devices.

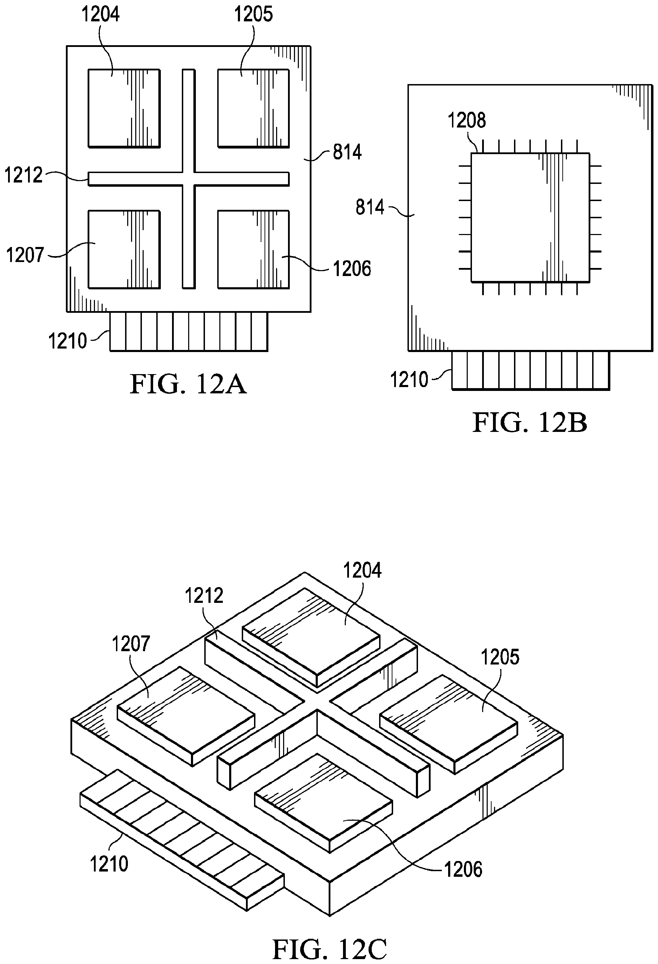

Referring then to Figures 12A, 12B, and 12C, daughterboard **814** is preferably a 2-sided PC board supporting optoelectronic devices **1204, 1205, 1206,** and **1207,** connector **1210,** and processor **1208.** Processor **1208** draws power from the battery and is supplied with an onboard memory that contains instructions for its operation. The optoelectrical devices are positioned in quadrants adjacent the proximal surface of the optical window. Each quadrant is separated by an optical opaque light baffle **1212.** In a preferred embodiment, the baffle is a "cross-shaped" PVC standoff, approximately 1-2 mm in height, coated with a reflective layer, such as $TiO_2$, on its exterior surface and bonded to the daughterboard with a suitable adhesive. Each of the optoelectronic devices is positioned to be perpendicular to and aligned with the optical axis of one lead channel in order to maximize either transmission or reception of light from an optical fiber, positioned in the lead channel. In a preferred embodiment, optoelectronic devices **1204, 1205, 1206,** and **1207** and light baffle **1212** may be integrated into one or more application specific integrated circuits (ASICs).

Connector **1210** links daughterboard **814** to the main circuit board of the IPG device. Processor **1208** is electrically connected to the optoelectronic devices through the daughterboard as required to communicate electrical signals to the processor.

In one embodiment, optoelectronic device **1204** is an optical emitter and optoelectronic devices **1205, 1206,** and **1207** are optical detectors.

In another embodiment, optoelectronic devices **1204,** and **1206** are optical emitters and optoelectronic devices **1205,** and **1207** are optical detectors.

The wavelengths of the emitters may range from visible red to infrared, or approximately 620-1700 nanometers. The emitter(s) may be either single wavelength or multiple wavelengths. For instance, the emitter could be a high-speed, single wavelength infrared emitting diode of 850 nm wavelength, such as part no. VSMY1850 available from Vishay Intertechnology, Inc. of Malvern, Pennsylvania. Alternatively, the emitter could be a multi-chip emitter, such as product no. MTMD6788594SMT6 available from Marktech Optoelectronics, Inc., of Latham, New York, which is capable of emitting wavelengths 670 nm, 770 nm, 810 nm, 850 nm, and 950 nm. Alternatively, an emitter and detector may be integrated into a single ASIC such as with the ADPD144RI from Analog Devices, Inc. of Norwood, Massachusetts.

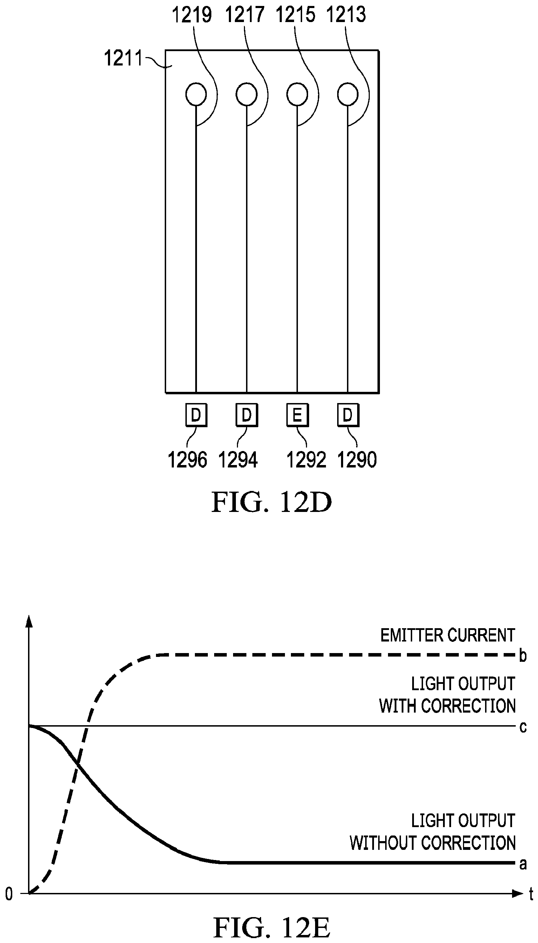

Referring to Figure 12D, a preferred embodiment of a coupling arrangement between optical leads and optical emitters in a surgical lead will be described. Emitter **1292** is optically coupled to central fiber **1215** of surgical lead **1211.** Detector **1290** is optically coupled to lead **1213** of surgical lead **1211.** Detectors **1294** and **1296** are connected to leads **1217** and **1219,** respectively.

Referring to Figure 12E, a graph showing light output from a side firing fiber of a surgical lead and input current to a corresponding emitter over time, will be described.

Light output over time is shown by the curve labeled "a". It can be seen that the light output of fiber **1215** degrades over time due to microfractures in the fiber and other degradation of optical components in the surgical lead. The decrease in optical performance of fiber **1215** is monitored over time by processor **1208** by reading the voltage signal from detector **1296,** which receives light from fiber **1215** reflected by the spinal cord. Processor **1208** is programmed to compensate for the degradation in light output by increasing the current to emitter **1204** according to curve "b". As can be seen, increasing the current to emitter **1204** maintains the light output of fiber **1215** at a consistent level shown by curve "c" as shown in the drawing.

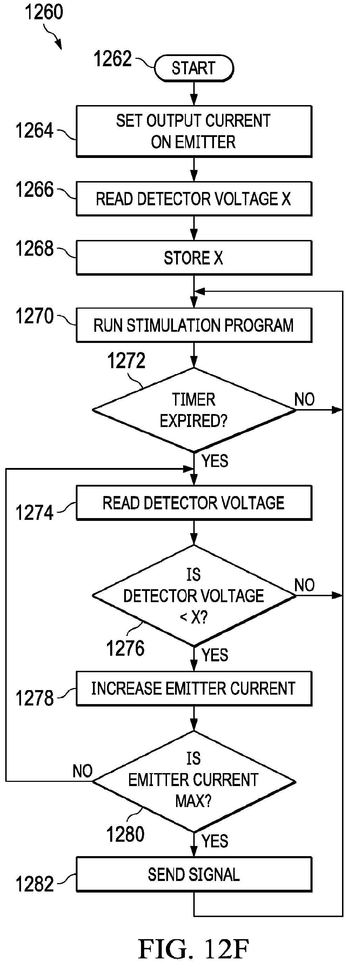

Referring to Figure 12F, a self-adjusting emitter current program for adjusting light output from an emitter fiber will be described. In a preferred embodiment, the program is a series of instructions that reside in the memory of processor **1208.**

At step **1262,** the program begins.

At step **1264,** the processor sets the output current to emitter **1292.** In a preferred embodiment, the emitter current is set to the minimum requirement to generate a readable signal at detectors **1290** and **1296.**

At step **1266,** the processor reads the voltage at detector **1294.** At step **1268,** the voltage level is stored in memory. At step **1270,** processor **1208** sends a signal to main circuit board **818** to initiate a stimulation program. The main circuit board responds by sending appropriate stimulation signals to the leads.

At step **1272,** processor **1208** determines whether or not a self-timer has expired. If so, the program proceeds to step **1274.** If not, the program returns to step **1270.**

At step **1274,** processor **1208** reads the detector voltage at detector **1294.**

At step **1276,** the processor compares the present value detector voltage to the stored detector voltage in memory. If the present value detector voltage is less than the stored detector voltage, then the process moves to step **1278.** If not, the program returns to step **1270.**

At step **1278,** processor **1208** increases the emitter current to emitter **1204.** In a preferred embodiment, the emitter current is increased by 1/100 of the maximum emitter current permitted.

At step **1280,** the processor determines whether or not the emitter current is set to the maximum allowed. If so, the program moves to step **1282.** If not, the program returns to step **1274.**

At step **1282,** the processor sends a signal to the main circuit board, which communicates it through the RF antenna to an external receiver, indicating that the maximum emitter current has been reached. The program then returns to step **1270.**

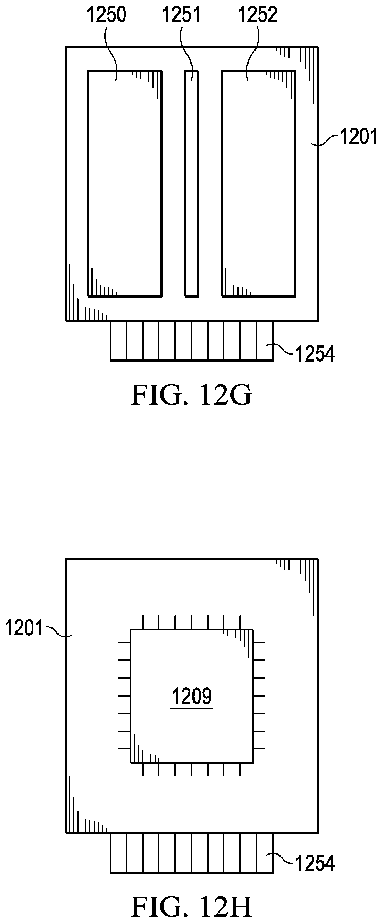

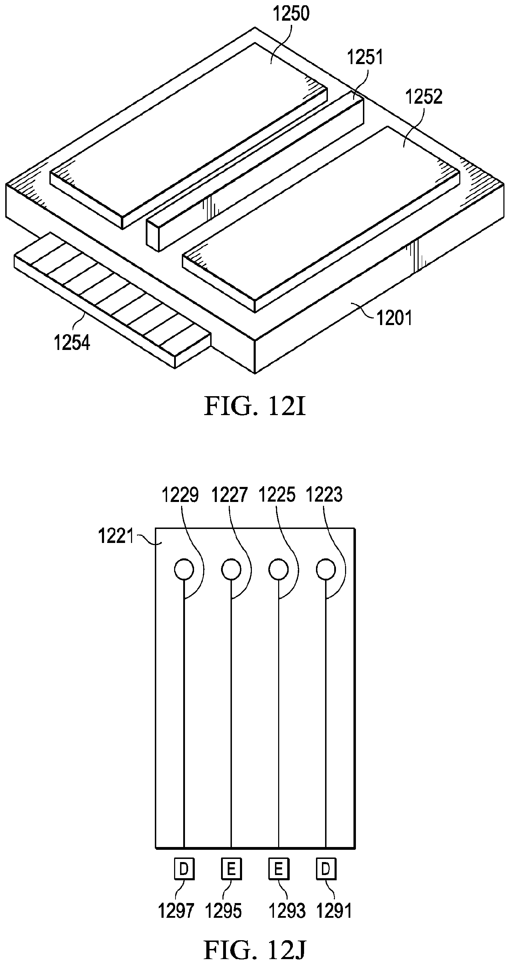

Referring then to Figures 12G, 12H, and 12I alternate embodiment of daughterboard **814** will be further described.

Daughterboard **1201** is a composite optoelectrical device comprised of optoelectronic devices **1250,** and **1252,** connector **1254,** and signal processor **1209.** The optoelectrical devices are positioned adjacent and parallel to the optical window. In a preferred embodiment, each optoelectronic device is separated by an optical opaque light baffle **1251.** In a preferred embodiment, baffle **1251** is a reflective or opaque rectangular PVC standoff bonded to the daughterboard, as previously described.

Connector **1254** links daughterboard **1201** to the main circuit board of the IPG device. Processor **1209** is electrically connected to the optoelectronic devices through the daughterboard as required and communicates external signals to the signal processor. The daughterboard communicates to the main circuit board through connector **1254.**

Referring then to Figure 12J, a preferred embodiment of a coupling arrangement between optical leads and optical emitters in a surgical lead will be described. Emitter **1293** is optically coupled to central fiber **1225** of signal lead **1221.** Detector **1291** is optically coupled to lead **1223** of signal lead **1221.** Emitter **1295** is optically coupled to central fiber **1227** and detector **1297** is connected to lead **1229.** In this configuration, dual optical reflectometry channels facilitate the stereoscopic detection of spinal cord position in the sagittal and coronal planes as previously described in U.S. Patent Nos. 8,239,038; 8,543,213; 9,132,273; 9,656,097 to Wolf II.

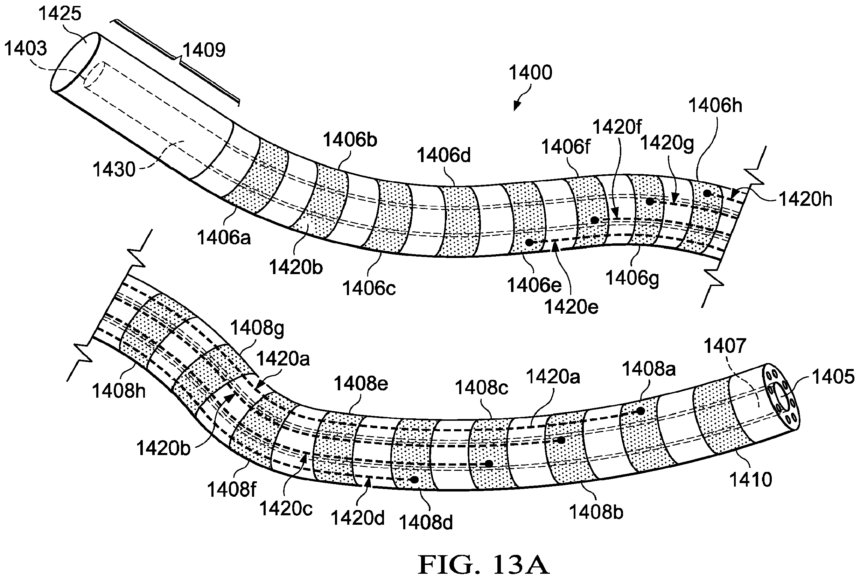

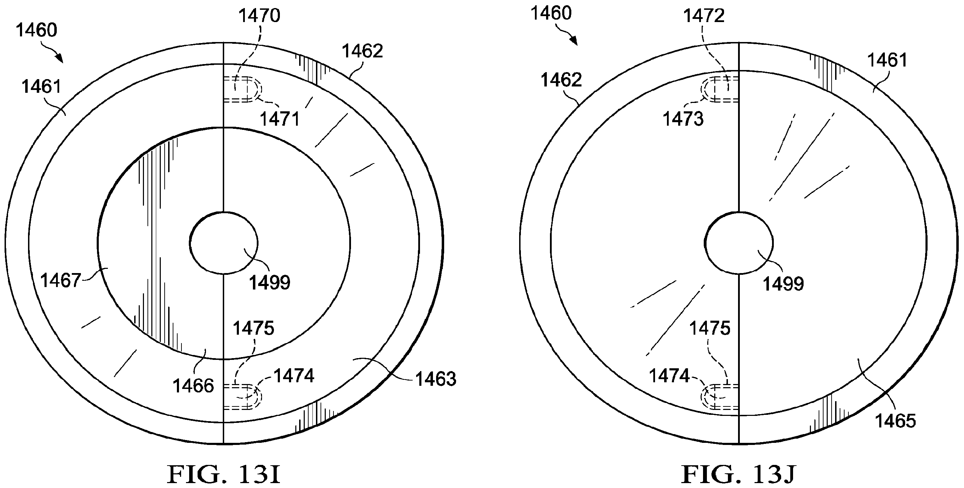

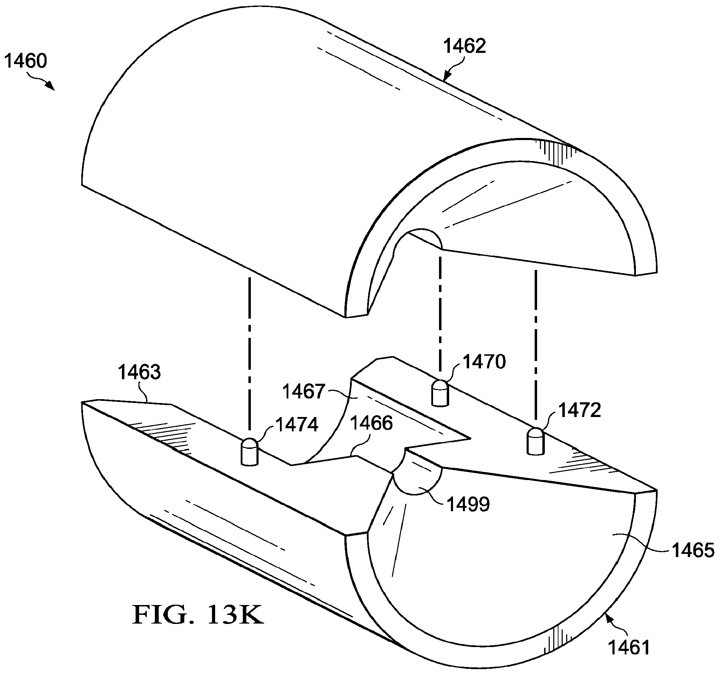

Referring to Figures 13A- 13G, a preferred embodiment of percutaneous lead **1400** is described.

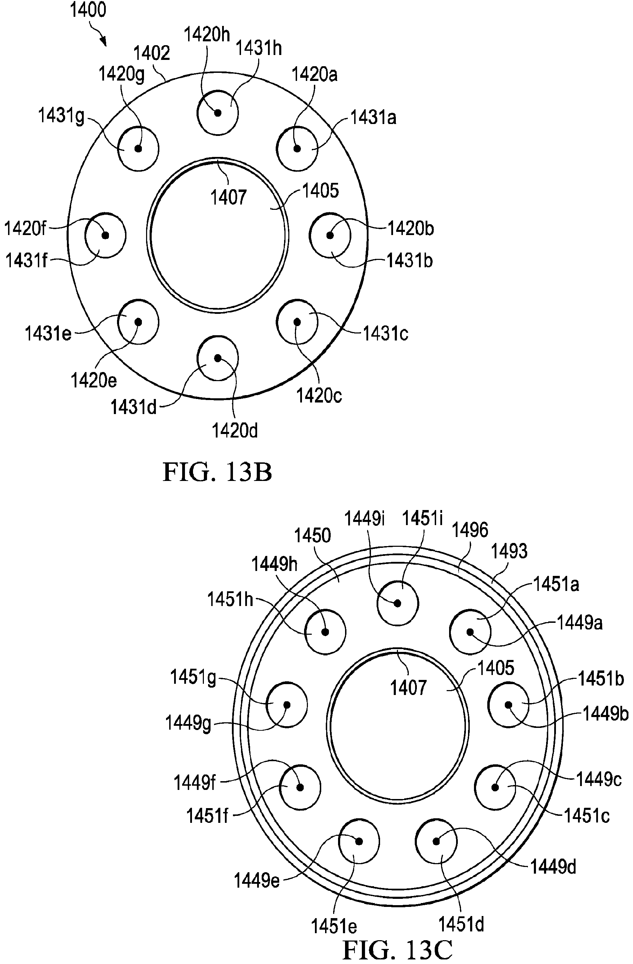

Referring then to Figures 13A and 13B, in a preferred embodiment of lead body **1402** is comprised of a generally hollow tube terminated by transmission window **1409.** In a preferred embodiment, the lead body is comprised of a flexible polymer such as Pellethane 55-D, or similar biocompatible polymer. The lead body is preferably a multi-lumen extrusion available from Zeus Industrial Products, Inc. of Orangeburg, South Carolina.

Transmission window **1409** is a hollow cylinder fused to the terminus of the flexible lead body enclosing diffuser cavity **1430.** In a preferred embodiment, the window is a suitable optically transparent material such as thermoplastic polyurethane. Transmission window **1409** is terminated by cap **1425.** Cap **1425** includes internally reflective surface **1403** which faces into diffuser cavity **1430.** In a preferred embodiment, the internally reflective surface is a titanium dioxide coating.

Stylet channel **1405** extends from the transmission window to the proximal end of the lead body. The stylet channel serves the dual purposes of housing a guide stylet for use during placement of the lead during surgery, and housing and optical fiber after surgery, as will be further described. In a preferred embodiment, stylet channel **1405** is lined with polytetrafluoroethylene (PTFE) lining **1407** which extends from the length of the lead body up to transmission window **1409.** The extremely low surface friction afforded by the carbon-fluorine bonds of the PTFE facilitates manual insertion of the stylet and the optical fiber. The lining does not extend into the diffuser cavity, where the side-firing segment of the optical transmission fiber resides, to enhance optical transmission.

Metallic anchor ring **1410** is positioned at the proximal end of the lead body. The anchor ring is generally cylindrical and is permanently affixed to the exterior of the lead body proximal to the lead contacts. Eight cylindrical proximal metallic contacts **1408a, 1408b, 1408c, 1408d, 1408e, 1408f, 1408g, 1408h** are fixed to the exterior of the lead body at even axial distances along the lead body and positioned to electrically contact the coil springs in the header.

In the same way, eight cylindrical distal metallic electrodes **1406a, 1406b, 1406c, 1406d, 1406e, 1406f, 1406g, 1406h** are provided at the distal end of the lead body. The distal lead contacts are each and permanently fixed to the exterior surface of the lead. The distal lead contacts are evenly spaced along the lead body proximal to the optical window.

The lead body further comprises eight radially oriented lumens **1431a, 1431b, 1431c, 1431d, 1431e, 1431f, 1431g** and **1431h.** Conductors **1420a, 1420b, 1420c, 1420d, 1420e, 1420f, 1420g, 1420h** are located in the lumens and extend from respective proximal contacts to distal electrodes. In a preferred embodiment the conductors are comprised of MP35N, or another conductive material similarly resistant to corrosion. Each of the conductors connects exactly one proximal contact to a single paired distal electrode.

Referring then to Figure 13C, a cross-sectional view of an alternate embodiment of lead body **1450,** is described.

Lead body **1450** comprises nine radially oriented lumens, **1449a, 1449b, 1449c, 1449d, 1449e, 1449f, 1449g, 1449h** and **1449i.** Conductors **1451a, 1451b, 1451c, 1451d, 1451e, 1451f, 1451g** and **1451h** and ground line **1451i** are located in the lumens. Ground line **1451i** extends from the proximal end of the lead body to the transmission window. Ground line **1451i** is electrically connected to anchor ring **1410.** When the anchor screw engages anchor ring **1410,** the ground lead is connected directly to the IPG ground either through the anchor block or through a ground connection through the header. The ground line may be used to supplement electrical shielding of the electrode array contacts for better MRI compatibility.

In another preferred embodiment, the lead body may incorporate non-metallic shielding layer **1496,** connected to ground line **1451i,** to further enhance MRI capability. In a preferred embodiment, the shielding layer is formed by carbon fibers infused into the surface of the lead body. In another preferred embodiment, low friction layer **1493,** such as PTFE, is included on the exterior of the lead body to aid in placement of the lead during surgery.

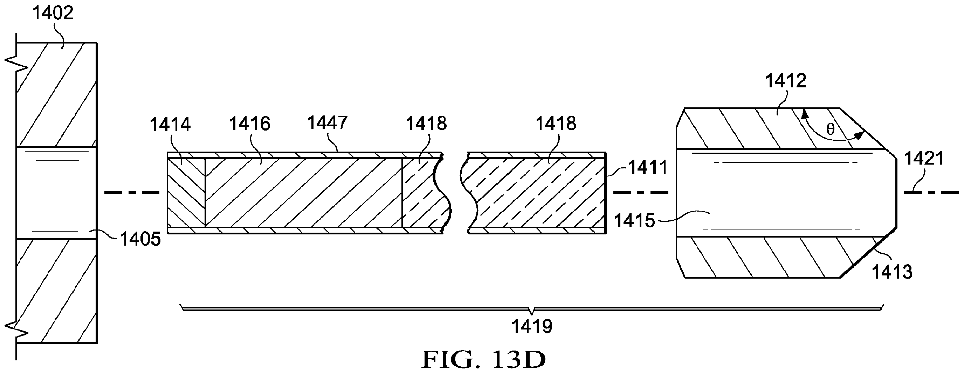

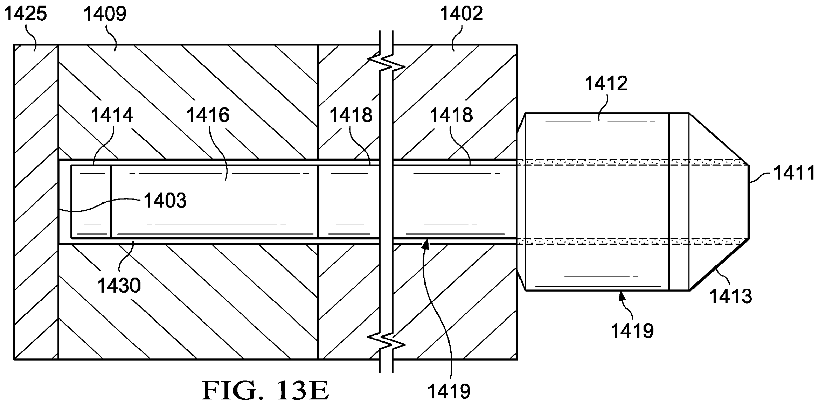

Referring then to Figures 13D and 13E, optical fiber subassembly **1419** includes ferrule **1412** and optical fiber **1418.**

Ferrule **1412** is generally a ceramic cylinder. Ferrule **1412** includes integrally formed alignment tip **1413.** Alignment tip **1413** is a chamfer formed in the ferrule with a chamfer angle of θ, preferably is between about 135° and 150°. In a preferred embodiment, chamfer angle θ matches the chamfer angle **735** of centering surface **716,** so that when the ferrule is mounted in the header there is an elastic compression of the ferrule by the polymer lead body. When mounted, the positive stop of the ferrule by ferrule centering surface **716** prevents pressure being applied to sealed optical window **718** by the fiber or the ferrule. Hole **1415** is centered in ferrule **1412** and extends through the length of the ferrule. In a preferred embodiment, the diameter of the hole closely matches the diameter of the optical fiber. In a preferred embodiment, ferrule **1412** is made of a polished ceramic, preferably zirconia, or other MRI compatible material.

Ferrule **1412** is positioned at the proximal end of optical fiber **1418**. Optical fiber **1418** includes end reflector **1414** and side-firing fiber segment **1416** at its distal end and polished optical tip **1411** at its proximal end. Optical fiber **1418** is preferably comprised of a polymethylmethacrylate core with a fluorocarbon cladding of about 250-400 micrometers in diameter. In a preferred embodiment, the fiber also includes a low friction layer **1447,** preferably comprised of PTFE. In use, the low friction layer aids in insertion of the fiber in the stylet lumen.

Optical fiber **1418** includes reflector **1414** at its distal end. The reflector prevents axial light emission from the fiber and improves radial dispersion of light. The reflector thereby improves optical signal strength and lowers power consumption. Ideally, the reflector is comprised of a titanium dioxide layer coated on the end of the fiber after it has been thermally polished.

Side-firing fiber segment **1416** is positioned in diffuser cavity **1430,** adjacent cap **1425,** and is typically about 5 mm in length. Side-firing fiber segment **1416** is formed by modification of the cladding of the optical fiber. The cladding may be modified by using femtosecond laser etching, mechanical abrasion, or an alternative method to achieve radial leakage of light.

Polished optical tip **1411** is positioned at the proximal end of the optical fiber. Polished optical tip **1411** is preferably a thermally polished surface perpendicular to the optical axis of the fiber. Optionally, a convex lens may be attached to the proximal end of the fiber to focus light into or out of the fiber, as will be further described.

Referring then to Figure 13E, optical fiber subassembly **1419** is positioned in stylet channel **1405.** The outer diameter of ferrule **1412** is less than the outer diameter of lead body **1402** but greater than the diameter of stylet channel **1405,** such that the lead body acts as a stop for the ferrule.

In one embodiment, optical fiber subassembly **1419** is placed in the stylet channel after surgical placement of the lead body *in vivo,* as will be further described.

In another embodiment, the fiber subassembly is prefabricated into the lead body. In this embodiment, the proximal end of the optical fiber is secured to the fiber by a suitable adhesive. One such suitable medical grade adhesive is preferably an optically transparent biocompatible epoxy seal, such as EPO-TEK® MED-353ND by Epoxy Technology, Inc. of Billerica, Massachusetts. In this case, polished optical tip **1411** is polished flush with alignment tip **1413.**

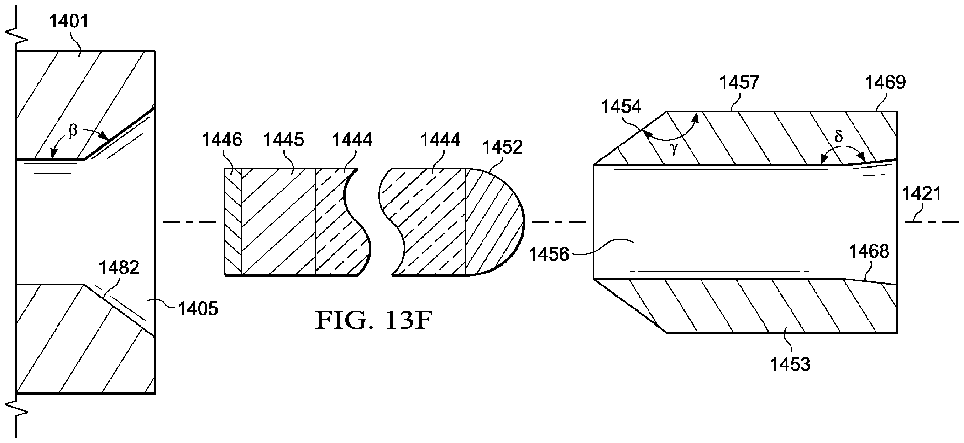

Referring then to Figure 13F, an alternate embodiment of optical fiber subassembly **1419** will be described.

Lead body **1401** includes stylet channel **1405** having an optical axis **1421.** The lead body incorporates proximal contacts, distal electrodes and electrical conductors, as previously described. Stylet channel **1405** is terminated with frustoconical flare **1482.** Frustoconical flare **1482** is coaxial with optical axis **1421.** The frustoconical flare has inclination angle β, which can range from about 135° to about 150°.

In a preferred embodiment, the lead channel has a diameter of about 15 - 20% greater than the optical fiber to allow the fiber to move within the channel. Optical fiber **1444** is preferably a plastic fiber, as previously described. Optical fiber **1444** includes end reflector **1446** and side-firing fiber segment **1445,** as previously described.

Optical fiber **1444** is proximally terminated by convex lens **1452.** Convex lens **1452** consists of a polished ceramic material, such as sapphire, fixed to the optical fiber using a suitable optically transparent adhesive. In another embodiment, the lens is formed integrally with the transmission fiber. In another embodiment, the optical fiber is polished flat and no lens is incorporated.

Collet **1457** includes collet body **1453.** Preferably, collet body **1453** is comprised of a ceramic material, such as Zirconia, or another MRI compatible material. Alignment tip **1454** is a chamfer integrally formed in the distal end of the collet body. Alignment tip **1454** forms angle of inclination γ of about 135°. In a preferred embodiment, angle γ matches angle β of frustoconical flare **1482.**

Collet body **1453** further includes cylindrical collet chamber **1456.** Collet chamber **1456** is coaxial with collet body **1453** and extends through alignment tip **1454.** In a preferred embodiment, the collet is bonded to optical fiber **1444** at the collet chamber.

Lens shield **1469** is integrally formed with the proximal end of collet body **1453.** It is designed to serve as a stop to prevent the optical fiber from impinging on the optical window of the IPG body. Lens shield **1469** further includes frustoconical lens opening **1468** ductedly connected with collet chamber **1456.** The frustoconical lens opening is coaxial with the collet chamber and has an angle of inclination δ of about 175° with the collet chamber. Frustoconical lens opening **1468** serves to focus light toward the optical window.

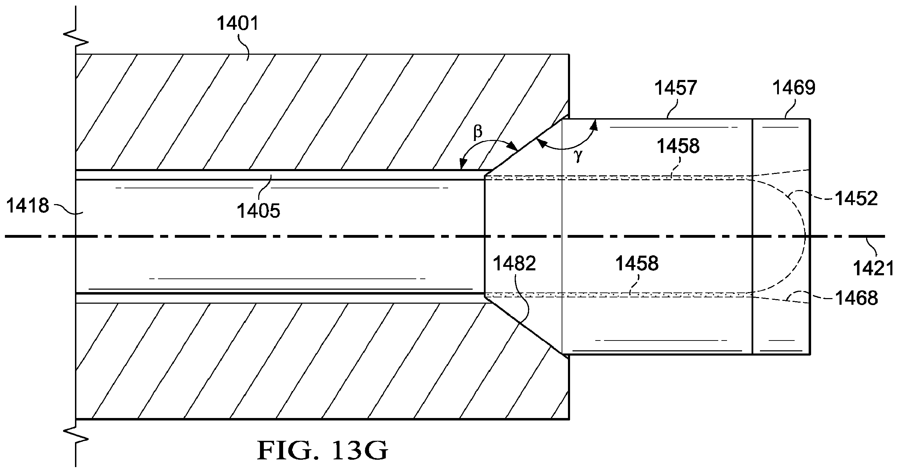

Referring then to Figure 13G, the proximal end of the optical fiber is shown positioned in collet chamber **1456.** Lens **1452** does not extend past lens shield **1469.** The fiber is fixed in the collet chamber by a suitable epoxy.

Optical fiber **1418** is positioned in stylet channel **1405** of lead body **1401**. Frustoconical flare **1482** serves to guide insertion of the optical fiber into the lead channel. The interface of frustoconical flare **1482** and alignment tip **1454** also serves to center optical fiber **1444** and lens **1452** on axis **1421**. The outer diameter of collet **1457** is less than the outer diameter of lead body **1401** but greater than the diameter of stylet channel **1405,** such that when the lead is inserted in the header, the lead body acts as a stop for the collet, such that the side firing fiber segment of the fiber is adjacent the optical window.

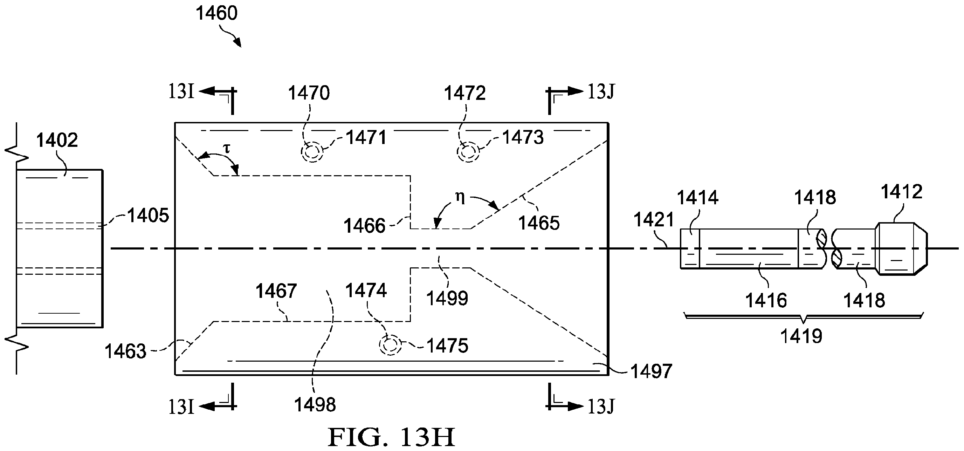

Referring then to Figure 13H - 13K, a preferred embodiment of optical threading assembly **1460** is described. Inserting the optical fiber into the stylet channel of the lead can be difficult due to the miniature size of the fiber and small diameter of the stylet channel. In the same way, during surgery, the replacement of the stylet in the stylet channel can be difficult. These difficulties are compounded by necessity for speed, manual dexterity and visual acuity. Improper insertion of the optical fiber can lead to damage to the fiber causing breakage or early degradation of the fiber. Likewise, improper stylet insertion can compromise the lead body. The optical fiber threading assembly solves these and other problems.

Optical threading assembly **1460** includes guide body **1497**. The guide body is a roughly 1 cm diameter cylinder and is comprised of thermoplastic. The assembly is preferably formed either using injection molding, or additive manufacturing. Other methods of manufacture will suffice. Guide body **1497** is generally cylindrical and is comprised of two opposing semicylinders, **1461** and **1462.**

Optical threading assembly **1460** includes frustoconical lead centering surface **1463** at its distal end. Frustoconical lead centering surface **1463** is coaxial with axis **1421**. Frustoconical lead centering surface **1463** is adjacent cylindrical alignment surface **1467**. Cylindrical alignment surface **1467** forms alignment cavity **1498**. Frustoconical lead centering surface **1463** forms an angle of inclination $\tau$ of about 135° with cylindrical alignment surface **1467.** The alignment cavity has a diameter generally equal to that of lead body **1402**. The alignment cavity is terminated with stop surface **1466**. Stop surface **1466** is a generally annular ring formed perpendicular to and coaxial with axis **1421.**

Adjacent to and ductedly connected with alignment cavity **1498,** is generally cylindrical fiber alignment duct **1499.** Fiber alignment duct **1499** is coaxial with axis **1421.** The diameter of the fiber alignment duct is generally the same as the diameter of stylet channel **1405** of lead body **1402.**

Fiber alignment duct **1499** is adjacent to and ductedly connected with frustoconical optical fiber centering surface **1465**. Frustoconical optical fiber centering surface **1465** forms an angle of inclination $\eta$ of about 135° with fiber alignment duct **1499**. Frustoconical optical fiber centering surface **1465** is coaxial with axis **1421.**

Semicylinder **1461** includes alignment pegs **1474, 1470,** and **1472**. Semicylinder **1462** includes alignment recesses **1475, 1471,** and **1473.** Alignment peg **1474** is diametrically opposed to alignment recess **1475.** Alignment peg **1470** is diametrically opposed to alignment recess **1471.** Alignment peg **1472** is diametrically opposed to alignment recess **1473.** The diameter of each of alignment recesses **1475, 1471,** and **1473** is such that alignment pegs **1474, 1470,** and **1472** can be secured by a press fit. Using the alignment pegs and recesses, the semicylinders may be easily assembled for use and then disassembled after use, as will be further described.

In use, optical threading assembly **1460** aligns lead body **1402** and optical fiber subassembly **1419** along axis **1421.** Lead body **1402** is aligned using frustoconical lead centering surface **1463** and held in position in alignment cavity **1498** by alignment surface **1467** and stop surface **1466**. Optical fiber subassembly **1419** is aligned using frustoconical centering surface **1465** and moved through fiber alignment duct **1499** and into the stylet channel of the lead body.

In the same way, a stylet may be positioned in the stylet channel in place of the optical fiber.

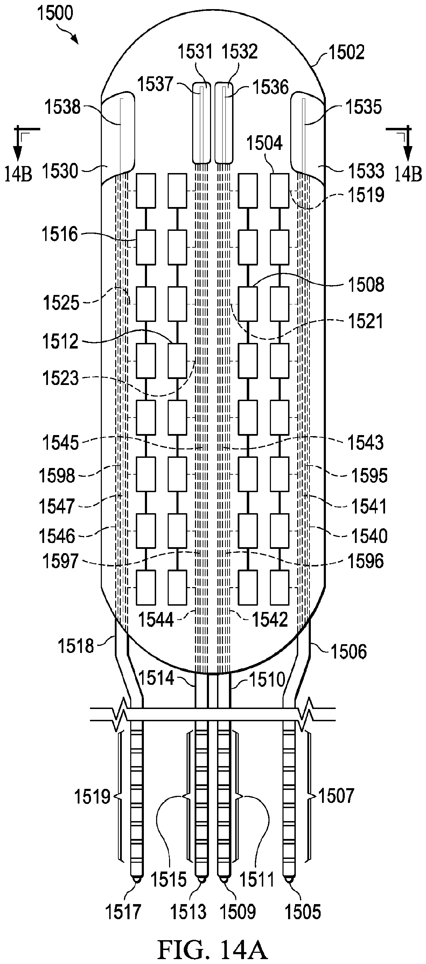

Referring then to Figures 14A - 15B, alternate embodiments for surgical leads are described.

Surgical leads may be configured with two or more multi-duct leads containing integrated optical fibers, depending upon the number of electrodes in the array and the desired number of optical reflectometry channels. The multi-duct leads may be organized into pairs of emitter leads and detector leads. Generally, a surgical lead configured with two multi-duct leads incorporate one optical reflectometry channel, while a surgical lead with four multi-duct leads incorporates two optical reflectometry channels. A surgical lead with two multi-duct leads with integrated optical fibers is capable of determining sagittal spinal cord position. Whereas a surgical lead with four multi-duct leads with integrated optical fibers is capable of determining sagittal and coronal spinal cord position.

Referring then to Figure 14A, a preferred embodiment of surgical lead **1500** is described.

Electrode array **1500** is comprised of integrated flexible panel **1502**. Panel **1502** is preferably of a medical grade inert polymer material, such as Pellethane 55-D. Flexible panel **1502** houses multi-duct leads **1506, 1510, 1514,** and **1518**. In a preferred embodiment, the multi-duct leads are sealed within the body of the flexible panel. Each of multi-duct leads **1506, 1510, 1514,** and **1518** includes a central lumen **1595, 1596, 1597** and **1598,** respectively. Each central lumen includes an optical transmission fiber **1541, 1543 1545** and **1547,** respectively. Each optical transmission fiber terminates at a distal end in a side firing optical fiber segment, **1535, 1536, 1537** and **1538,** respectively. The side firing optical fiber segments are constructed, as previously described. Each side firing optical fiber segment is positioned adjacent distally positioned optical window **1533, 1532, 1531** and **1530,** respectively. In a preferred embodiment, each of the optical windows is an optically transparent segment of the polymer comprising flexible panel **1502**. By placing the optical windows and the side firing segment at the most distal portion of the panel, there are no metallic components such as electrodes or connections to interfere with radial dispersion of light, while keeping the optical sensing region proximate the electrode arrays. This improves optical signal strength and consequently lowers power consumption. In an alternate embodiment, the optical windows are placed parallel to and adjacent columns of the electrode arrays.

Panel **1502** further comprises electrode arrays **1504, 1508, 1512,** and **1516** positioned adjacent multi-duct leads **1506, 1510, 1514** and **1518,** respectively. In a preferred embodiment, each of electrode arrays **1504, 1508, 1512,** and **1516** include eight electrodes embedded in the panel and having an exposed face external to the panel. Each of multi-duct leads **1506, 1510, 1514** and **1518** incorporate eight electrical conductors that extend the length of the panel and the multi-duct leads, as previously described. Each electrode is connected through the conductors in the lead body to exactly one lead contact. Electrode array **1504** is connected to lead contacts **1507.** Electrode array **1508** is connected to lead contacts **1511.** Electrode array **1512** is connected to lead contacts **1515.** Electrode array **1516** is connected to lead contacts **1519.** In a preferred embodiment, the electrodes are comprised of platinum-iridium alloy (nominally 90%/10% to 80%/20%).

Each of multi-duct leads **1506, 1510, 1514** and **1518** terminates at ferrules **1505, 1509, 1513** and **1517,** respectively. In a preferred embodiment, the ferrules are bonded to the fibers, as previously described.

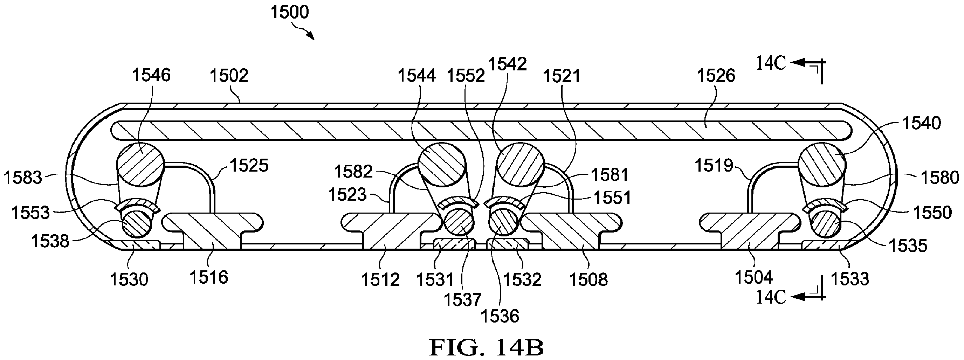

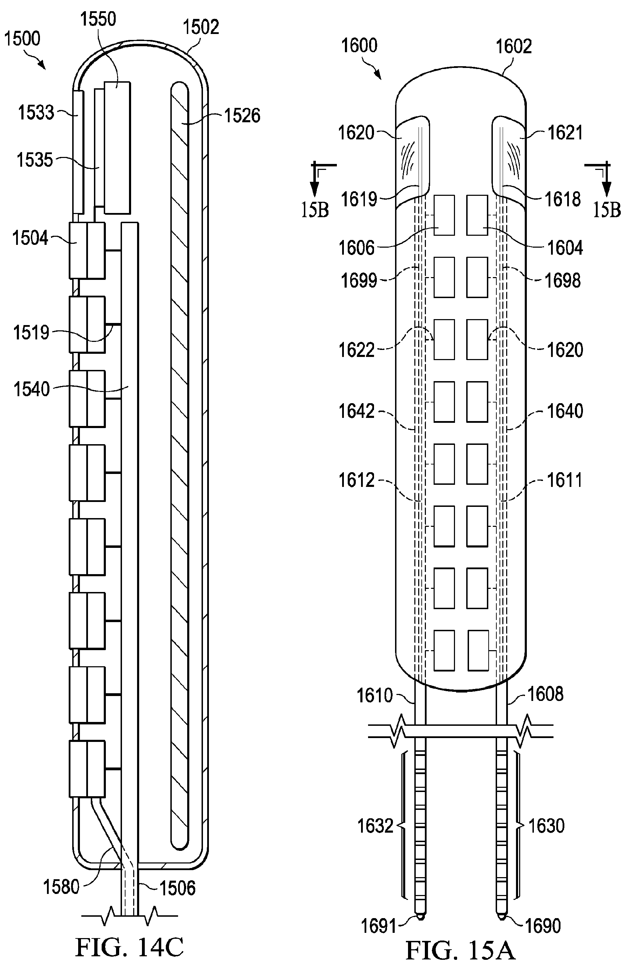

Referring then to Figures 14B and 14C, each of electrode arrays **1504, 1508, 1512** and **1516** is connected through electrical connections **1519, 1521, 1523** and **1525** to one of conductor bundles **1540, 1542, 1544** and **1546,** respectively. The conductor bundles contain the individual conductors, radially separated, as previously described. The side firing fiber segments separate from the multi-duct leads and away from the conductor bundles in manifolds **1580, 1581, 1582** and **1583,** respectively.

Panel **1502** includes light reflectors **1550, 1551, 1552** and **1553,** adjacent side firing optical fiber segments **1535, 1536, 1537,** and **1538,** respectively. The light reflectors are preferably semicylindrical or parabolic, and flexible. In a preferred embodiment, light reflectors **1550, 1551, 1552** and **1553** are comprised of a non-conductive material polymer, such as Pellethane-55D, coated with a non-conductive reflective surface, such as titanium dioxide. This material operates at the desired wavelength from red to infrared and may be applied as a paint or film. The reflections improve optical efficiency by redirecting radially produced light from the emitter fiber segments toward the optical windows, or, alternatively, reflecting incoming light from the optical windows and into the detector fiber segments.

Panel **1502** is further comprised of lattice shield **1526.** Lattice shield **1526** is comprised of a generally flat flexible film and interdigitates with the polymeric material of the lead body. In a preferred embodiment, lattice shield **1526** is coated with a reflective material, such as titanium dioxide ($TiO_2$) adjacent the optical fibers. Lattice shield is contained within the panel adjacent each of conductor bundles **1540, 1542, 1544** and **1546,** and generally extends the length of the panel.

In one embodiment, the lattice shield may be comprised of an electrically conductive material, such as carbon nanofibers, and operates as a heat-sink to draw heat away from the electrode contact arrays and disperse it dorsally. In another embodiment, leads **1518, 1514, 1510,** and **1506** each include a ground, as shown in Figure 13C. Ground line **1451i** connects lattice shield **1526** with the anchor ring located at the proximal end of the lead. The anchor ring is connected to the IPG ground. This configuration provides optimal electrical shielding for MRI compatibility.

Referring then to Figure 15A, a preferred embodiment of surgical lead **1600** is described.

Surgical lead **1600** is comprised of integrated flexible panel **1602.** In a preferred embodiment, flexible panel **1602,** is a medical grade inert flexible polymeric material, as previously described. Integrated within the flexible panel are multi-duct leads **1608** and **1610.** Each of multi-duct leads **1608** and **1610** includes central lumen **1698** and **1699,** respectively. The central lumens include optical fibers **1611** and **1612,** respectively. Each optical fiber terminates in a side firing optical fiber segment **1618** and **1619,** respectively. The side firing optical segments are constructed as previously described. Each side firing optical segment is positioned adjacent an optical window **1621** and **1620,** respectively. In a preferred embodiment, each of the optical windows is an integrally formed optically transparent region of flexible panel **1602.**

Panel **1602** further comprises electrode arrays **1604** and **1606.** Each of electrode arrays **1604** and **1606** includes eight electrodes, embedded in the surface of panel **1602,** having an exposed face exterior to the panel. In a preferred embodiment, the electrodes are a platinum-iridium alloy. Each of the electrodes is connected through the conductors in the multi-duct lead bodies to exactly one lead contact, as previously described. Electrode array **1604** is connected to lead contacts **1630.** Electrode array **1606** is connected to lead contacts **1632.**

Each of multi-duct leads **1608** and **1610,** terminates at ferrules **1690** and **1691,** respectively. The ferrules are attached and bonded to the optical fibers, as previously described.

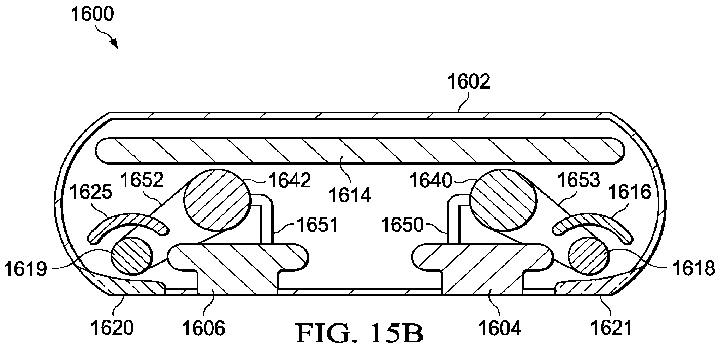

Referring then to Figure 15B, the conductors in the multi-duct lead bodies separate from side firing optical fiber segment **1618** and **1619** in optical manifolds **1653** and **1652,** respectively. Each of electrode arrays **1604** and **1606** is connected through electrical connections **1650** and **1651** to the conductors in one of conductor bundles **1640** and **1642,** respectively.

Panel **1602** is further comprised of lattice shield **1614.** Lattice shield **1614** is comprised of a generally flat flexible film that is integrally formed with both the flexible panel. As previously described, the lattice shield may be coated with reflective material adjacent the optical fibers. The lattice shield may be connected to a ground contact for further connection to the IPG ground, as previously described.

Panel **1602** further comprise reflectors **1616** and **1625,** positioned adjacent side firing optical fiber segments **1618** and **1619,** respectively. In preferred embodiments, the reflectors are generally semicylindrical or are parabolic. In another embodiment, the reflectors may be flat flexible panels. The reflectors serve to aid in the reflection of light emitted from a side firing fiber segment out through an optical window or to focus incoming light through optical windows and back into the fiber for transmission to a detector within the IPG.

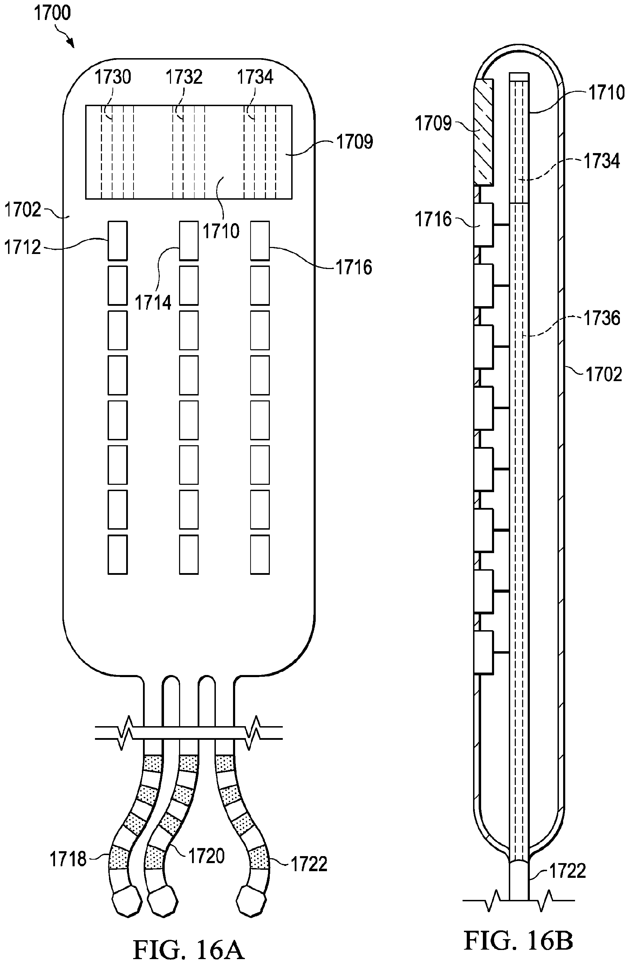

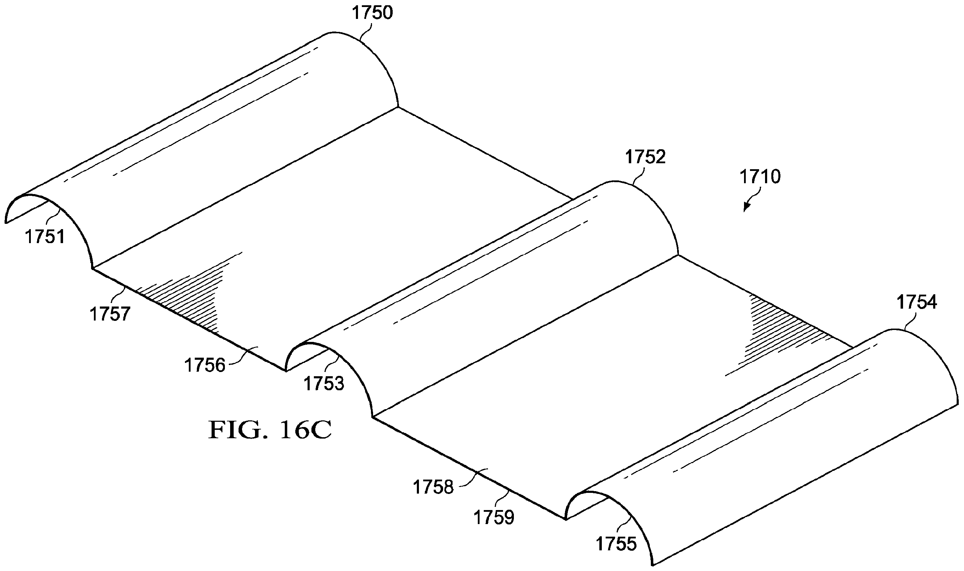

Referring to the Figure 16A, 16B and 16C, an alternate embodiment of surgical lead **1700** will be described. Integrated panel **1702** is generally flat, polymeric and rectangular, as previously described. Integrated within integrated panel **1702** are multi-duct leads **1718, 1720** and **1722** with optical fiber **1736,** as previously described. Each of the multi-duct leads have proximal electrical contacts which are individually connected to electrode arrays **1712, 1714** and **1716** through conductors, as previously described. Integrated panels **1702** further comprises optical window **1709** integrated into the panel adjacent side firing fiber segments **1730, 1732** and **1734,** as previously described.

Composite reflector **1710** is comprised a plurality of alternating parabolic surfaces, such as parabolic surfaces **1750, 1752** and **1754,** and flat interstitial surfaces such as surfaces **1756** and **1758.** The parabolic surfaces and the flat surfaces are preferably comprised of a flexible inert plastic with sufficient rigidity to sustain moderate bending. In a preferred environment, polyvinyl chloride is used. Internal surfaces **1751, 1753** and **1755** of the parabolic surfaces and internal surfaces **1757** and **1759** of the flat surfaces all are coated with a reflective material such as titanium dioxide. Internal surfaces **1751, 1753** and **1755** are positioned adjacent side firing fiber segments **1730, 1732** and **1734** and serve to function as previously described.

In another preferred embodiment, composite reflector **1710** is grounded to the IPG case, through a conductor in one of the multi-duct leads, as previously described.

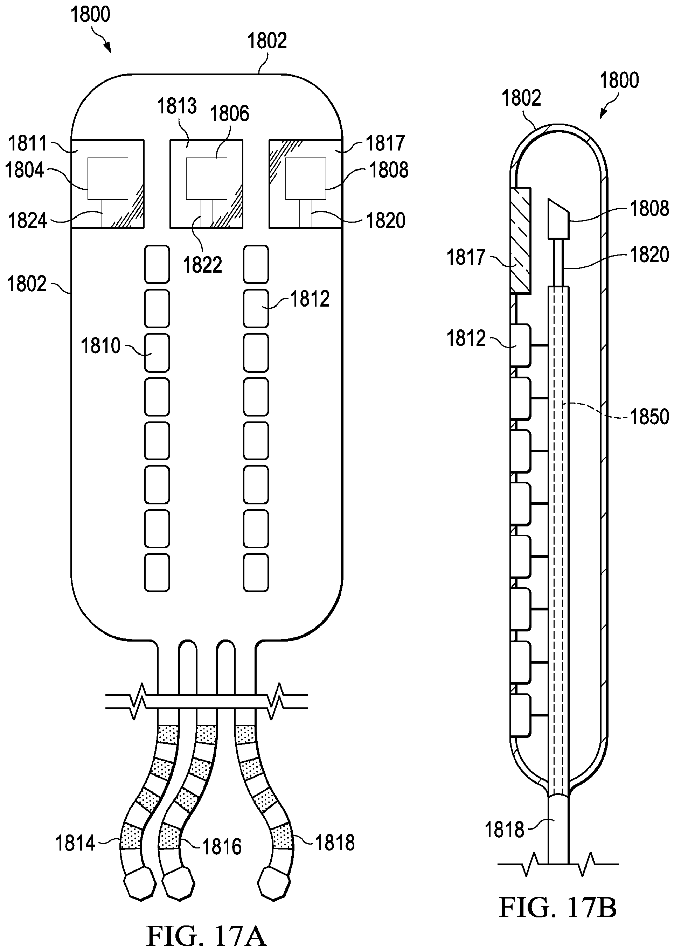

Referring then to Figures 17A and 17B an alternate embodiment of surgical lead **1800** will be further described.

Integrated panel **1802** generally comprises a flat, polymeric and rectangular, as previously described. Integrated panel **1802** include electrode arrays **1810** and **1812** connected to multi-duct leads **1814** and **1818,** as previously described. Integrated panel **1802** further comprise multi-duct leads **1814, 1816** and **1818** integrally formed, as previously described. Each of multi-duct leads **1814, 1816** and **1818** includes optical fiber **1850** with side firing fiber segments **1820, 1822,** and **1824.** Each optical fiber **1850** is positioned to terminate in a right-angle prism, such as right-angle prism **1804, 1806,** and **1808.** The right-angle prisms are positioned to direct light to an optical fiber from optical windows **1811, 1813,** and **1817,** or from the optical fiber to an optical window, as the case may be.

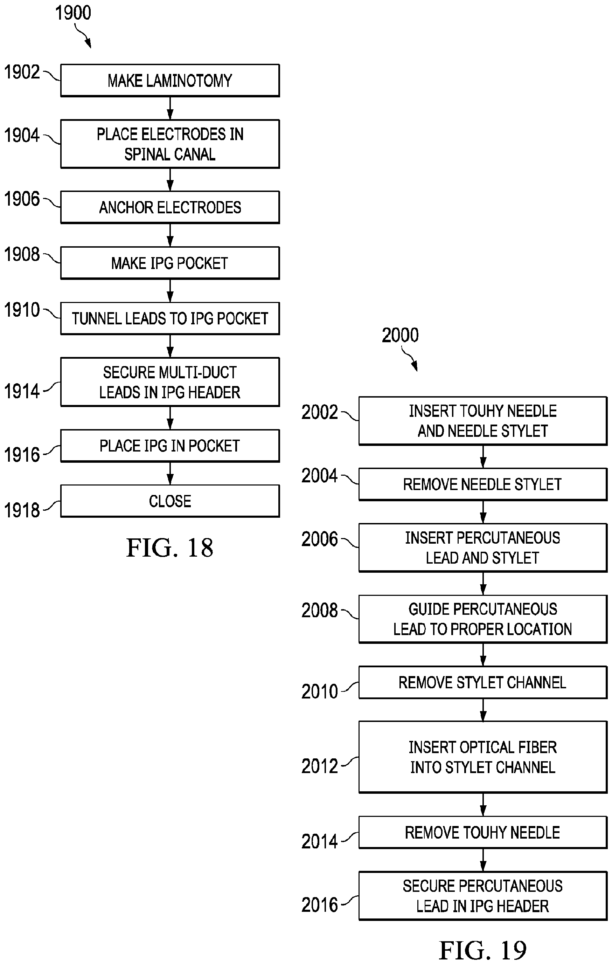

Referring then to Figure 18, method **1900** for the placement of a surgical lead, will be described.

At step **1902,** a laminotomy is conducted at the segmental level corresponding to the somatotopic distribution of the patient's pain.

At step **1904,** electrodes are placed in the spinal canal. Typically, the electrodes are placed in the dorsal epidural space by manually inserting the electrode array in the laminotomy cavity.

At step **1906,** the electrodes are anchored to the fascia, ligament or the adjacent bone.

At step **1908,** an incision is made for the IPG.

At step **1910** the leads are tunneled subcutaneously from the electrode insertion site to the IPG pocket.

At step **1914,** the multi-duct leads are secured in the IPG header. If the fibers are not secured in the multi-duct leads, then they may be inserted and secured at this step, as will be further described. In practice, the lead body, including the fiber subassembly, is threaded into the appropriate lead channel bringing the proximal lead contacts into electrical contact with the canted coil springs, of the lead channel. The multi-duct lead is advanced in the lead channel until the multi-duct lead body encounters frustoconical centering surface **724,** which guides it along cylindrical alignment surface **726,** until it engages stop surface **730** in anchor ring chamber **728.** Simultaneously, the ferrule is advanced into alignment cylinder **712** until it encounters ferrule centering surface **716.** Ferrule centering surface **716** aligns the optical fiber in buffer gap **734** and with the optical window in the IPG casing, adjacent the composite optoelectronic device **740.** The multi-duct lead is secured in the lead channel by advancing anchor screw **708,** using a torque limited ratchet, until it engages anchor ring **1410.**

At step **1916** the IPG is placed in the pocket.

At step **1918,** the procedure is ended.

Referring to Figure 19, preferred method **2000** of placement of a percutaneous lead will be described.

At step **2002,** a Touhy needle and needle stylet are inserted into spinal canal at the appropriate segmental level.

At step **2004,** the needle stylet is removed from the lumen of the Touhy needle.

At step **2006,** the percutaneous lead with included stylet guide wire is inserted into the bore of the Touhy needle.

At step **2008,** the percutaneous lead is guided to the proper location in the spinal canal using the stylet guide wire, under fluoroscopy.

At step **2010,** the stylet guide wire is removed from the stylet channel.

At step **2012,** the optical fiber is inserted into the stylet channel, as previously described.

At step **2014,** the Touhy needle is removed, while holding the lead in place.

At step **2016,** the proximal end of the percutaneous lead is secured in the IPG header, as previously described.

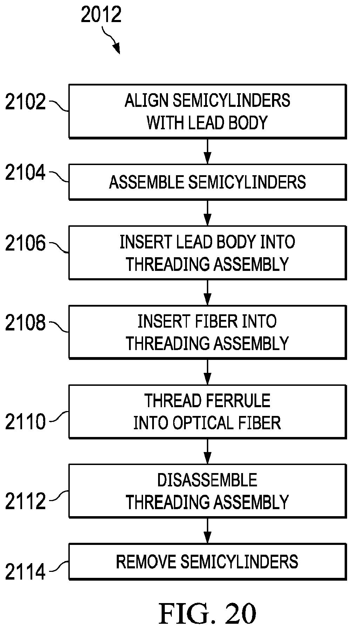

Referring then to Figure 20, step **2012** of securing the optical fiber in a stylet channel of a lead, will be further described.

At step **2102,** semicylinders **1461** and **1462,** are aligned with the percutaneous lead body. At step **2104,** semicylinders **1461** and **1462** are assembled by press fit. At step **2106,** the proximal end of the percutaneous lead body is inserted into the alignment cavity of the threading assembly, guided by frustoconical lead centering surface **1463.** Alignment surface **1467** aligns the multi-duct lead body with alignment cavity **1498.**

At step **2108,** optical fiber **1418** is inserted into fiber alignment duct **1499,** guided by frustoconical optical fiber centering surface **1465.** The optical fiber is then inserted into stylet channel **1405** of multi-duct lead body **1402,** of the threading assembly.

At step **2110,** ferrule **1412** is threaded onto optical fiber **1418.**

In an alternate embodiment, a stylet may be placed in the fiber alignment duct and the stylet channel at this step. If so, the method concludes here.

At step **2112,** the threading assembly is disassembled.

At step **2114,** the semicylinders are removed from the assembled lead body and optical fiber.

**Claims**

**1.** A surgical lead (1500, 1700, 1800) comprising:

a flexible panel (1502);
a set of electrode arrays embedded (1504, 1608, 1512, 1516) in the flexible panel;
a set of leads (1506, 1510, 1514, 1518) integrally formed with the flexible panel;
a set of lumens (1595, 1596, 1697, 1598);
at least one lumen of the set of lumens resident in at least one lead of the set of leads;
a set of conductors in the at least one lead of the set of leads;
a set of contacts (1507, 1511, 1515, 1519), attached to the at least one lead of the set of leads;
at least one conductor of the set of conductors, connected between a contact of the set of contacts and an electrode array of the set of electrode arrays;
a set of window portals (1533, 1532, 1531, 1530), in the flexible panel, adjacent to and distal from the set of electrode arrays;
a set of optical fibers (1541, 1543, 1545, 1547);
a set of side firing sections (1535, 1536, 1537, 1538);
at least one optical fiber of the set of optical fibers having at least one side firing section of the set of side firing sections;
at least one optical fiber of the set of optical fibers resident in the at least one lumen of the set of lumens;
the at least one side firing section positioned adjacent a window portal of the set of window portals;
a set of ferrules (1505, 1509, 1513, 1517); and,
at least one ferrule, of the set of ferrules, positioned on the at least one optical fiber of the set of optical fibers.

**2.** The surgical lead of claim 1 further comprising:

a set of reflectors (1550, 1551, 1552, 1553); and,
at least one reflector, of the set of reflectors, adjacent to the at least one side firing section of the set of side firing sections.

**3.** The surgical lead of claim 1 further comprising:

a set of diffusion chambers;
at least one diffusion chamber of the set of diffusion chambers connected to the at least one lead of the set of leads; and,
the at least one side firing section of the set of side firing sections, resident in the at least one diffusion chamber of the set of diffusion chambers.

**4.** The surgical lead of claim 3 further comprising:

a set of internally reflective surfaces; and,
at least one internally reflective surface of the set of internally reflective surfaces located at a distal end of the
at least one diffusion chamber of the set of diffusion chambers.

5. The surgical lead of claim 2, wherein at least one reflector of the set of reflectors is comprised of a non-metallic material or is non-metallic substrate coated with $TiO_2$, or wherein the set of reflectors is integrated into a reflective panel, or wherein a reflector of the set of reflectors further comprises a prism directed toward a window portal of the set of window portals.

6. The surgical lead of claim 1 wherein the set of conductors is arranged in an axially aligned radial pattern.

7. The surgical lead of claim 1 further comprising:
a generally planar heatshield, adjacent the set of leads.

8. The surgical lead of claim 1 further comprising:
a generally planar flexible lattice shield (1526), adjacent the set of leads.

9. The surgical lead of claim 8, wherein the lattice shield is a carbon fiber material, or wherein the lattice shield is grounded to an implantable pulse generator by a conductor of the set of conductors, or wherein the lattice shield further comprises a non-metallic reflective surface.

10. The surgical lead of claim 8, wherein the lattice shield is grounded to an implantable pulse generator by a conductor of the set of conductors and at least one lead of the set of leads further comprises a non-metallic conductive layer, grounded to an implantable pulse generator.

11. The surgical lead of claim 1 wherein an optical fiber of the set of optical fibers is a polymethylmethacrylate material.

12. The surgical lead of claim 1 wherein the set of leads further comprises:

a first pair of leads for transmission of a first light signal; and,
a second pair of leads for reception of a second light signal, or wherein the set of leads further comprises:

a first lead for transmission of a first light signal; and,
a second lead for reception of a second light signal.

13. The surgical lead of claim 1 wherein the at least one ferrule of the set of ferrules is bonded to the at least one optical fiber of the set of optical fibers.

**Patentansprüche**

1. Chirurgische Leitung (1500, 1700, 1800), umfassend:

eine flexible Platte (1502);
einen Satz von Elektrodenanordnungen, die in die flexible Platte eingebettet (1504, 1608, 1512, 1516) sind;
einen Satz von Leitungen (1506, 1510, 1514, 1518), die mit der flexiblen Platte integral ausgebildet sind;
einen Satz von Lumen (1595, 1596, 1697, 1598);
mindestens ein Lumen des Satzes von Lumen, der sich in mindestens einer Leitung des Satzes von Leitungen befindet;
einen Satz von Leitern in mindestens einer Leitung des Satzes von Leitungen;
einen Satz von Kontakten (1507, 1511, 1515, 1519), der an mindestens einer Leitung des Satzes von Leitungen angebracht ist;
mindestens ein Leiter des Satzes von Leitern, der zwischen einem Kontakt des Satzes von Kontakten und einer Elektrodenanordnung des Satzes von Elektrodenanordnungen verbunden ist;
einen Satz von Fensterportalen (1533, 1532, 1531, 1530), in der flexiblen Platte, angrenzend an und distal zu dem Satz von Elektrodenanordnungen;
einen Satz von optischen Fasern (1541, 1543, 1545, 1547);
einen Satz von seitlichen Zündabschnitten (1535, 1536, 1537, 1538);

mindestens eine optische Faser des Satzes von optischen Fasern mindestens einen seitlichen Zündabschnitt des Satzes von seitlichen Zündabschnitten aufweist;

mindestens eine optische Faser des Satzes von optischen Fasern, die sich in mindestens einem Lumen des Satzes von Lumen befindet;

wobei mindestens ein seitlicher Zündabschnitt angrenzend an ein Fensterportal des Satzes von Fensterportalen positioniert ist;

einen Satz von Aderendhülsen (1505, 1509, 1513, 1517); und

mindestens eine Aderendhülse, des Satzes von Aderendhülsen, die auf der mindestens einen optischen Faser des Satzes von optischen Fasern positioniert ist.

2. Chirurgische Leitung nach Anspruch 1, ferner umfassend:

ein Satz von Reflektoren (1550, 1551, 1552, 1553); und

mindestens ein Reflektor, des Satzes von Reflektoren, angrenzend an mindestens einen seitlichen Zündabschnitt des Satzes von seitlichen Zündabschnitten.

3. Chirurgische Leitung nach Anspruch 1, ferner umfassend:

ein Satz von Diffusionskammern;

mindestens eine Diffusionskammer des Satzes von Diffusionskammern, die mit mindestens einer Leitung des Satzes von Leitungen verbunden ist; und

der mindestens eine seitliche Zündabschnitt des Satzes von seitlichen Zündabschnitten, der sich in der mindestens einen Diffusionskammer des Satzes von Diffusionskammern befindet.

4. Chirurgische Leitung nach Anspruch 3, ferner umfassend:

ein Satz von intern reflektierenden Oberflächen; und

mindestens eine intern reflektierende Oberfläche des Satzes von intern reflektierenden Oberflächen sich an einem distalen Ende der mindestens einen Diffusionskammer des Satzes von Diffusionskammern befindet.

5. Chirurgische Leitung nach Anspruch 2, wobei mindestens ein Reflektor des Satzes von Reflektoren aus einem nichtmetallischen Material besteht oder ein mit $TiO_2$ beschichtetes nichtmetallisches Substrat ist, oder wobei der Satz von Reflektoren in eine reflektierende Platte integriert ist, oder wobei ein Reflektor des Satzes von Reflektoren ferner ein Prisma umfasst, das auf ein Fensterportal des Satzes von Fensterportalen gerichtet ist.

6. Chirurgische Leitung nach Anspruch 1, wobei der Satz von Leitern in einem axial ausgerichteten radialen Muster eingerichtet ist.

7. Chirurgische Leitung nach Anspruch 1, ferner umfassend:
ein im Allgemeinen ebener Hitzeschutz angrenzend an den Satz von Leitungen.

8. Chirurgische Leitung nach Anspruch 1, ferner umfassend:
eine im Wesentlichen ebene flexible Gitterabschirmung (1526) angrenzend an den Satz von Leitungen.

9. Chirurgische Leitung nach Anspruch 8, wobei die Gitterabschirmung ein Kohlenstofffasermaterial ist oder wobei die Gitterabschirmung durch einen Leiter des Satzes von Leitern an einem implantierbaren Impulsgenerator geerdet ist oder wobei die Gitterabschirmung ferner eine nichtmetallische reflektierende Oberfläche umfasst.

10. Chirurgische Leitung nach Anspruch 8, wobei die Gitterabschirmung durch einen Leiter des Satzes von Leitern mit einem implantierbaren Impulsgenerator geerdet ist und mindestens eine Leitung des Satzes von Leitungen ferner eine nichtmetallische leitfähige Schicht umfasst, die mit einem implantierbaren Impulsgenerator geerdet ist.

11. Chirurgische Leitung nach Anspruch 1, wobei eine optische Faser des Satzes von optischen Fasern ein Polymethylmethacrylatmaterial ist.

12. Chirurgische Leitung nach Anspruch 1, wobei der Satz von Leitungen ferner umfasst:

ein erstes Paar von Leitungen für eine Übertragung eines ersten Lichtsignals; und

ein zweites Paar von Leitungen für einen Empfang eines zweiten Lichtsignals, oder wobei der Satz von Leitungen ferner umfasst:

eine erste Leitung für die Übertragung eines ersten Lichtsignals; und
eine zweite Leitung für den Empfang eines zweiten Lichtsignals.

13. Chirurgische Leitung nach Anspruch 1, wobei die mindestens eine Aderendhülse des Satzes von Aderendhülsen an die mindestens eine optische Faser des Satzes von optischen Fasern gebunden ist.

**Revendications**

1. Fil chirurgical (1500, 1700, 1800) comprenant :

un panneau flexible (1502) ;
un ensemble de réseaux d'électrodes intégrées (1504, 1608, 1512, 1516) dans le panneau flexible ;
un ensemble de fils (1506, 1510, 1514, 1518) formés d'un seul tenant avec le panneau flexible ;
un ensemble de lumières (1595, 1596, 1697, 1598) ;
au moins une lumière de l'ensemble de lumières résidant dans au moins un fil de l'ensemble de fils ;
un ensemble de conducteurs dans l'au moins un fil de l'ensemble de fils ;
un ensemble de contacts (1507, 1511, 1515, 1519), fixés à l'au moins un fil de l'ensemble de fils ;
au moins un conducteur de l'ensemble de conducteurs, connecté entre un contact de l'ensemble de contacts et un réseau d'électrodes de l'ensemble de réseaux d'électrodes ;
un ensemble de portails de fenêtre (1533, 1532, 1531, 1530), dans le panneau flexible, adjacents à et distaux de l'ensemble de réseaux d'électrodes ;
un ensemble de fibres optiques (1541, 1543, 1545, 1547) ;
un ensemble de sections latérales de déclenchement (1535, 1536, 1537, 1538) ;
au moins une fibre optique de l'ensemble de fibres optiques ayant au moins une section latérale de déclenchement de l'ensemble de sections latérales de déclenchement ;
au moins une fibre optique de l'ensemble de fibres optiques résidant dans l'au moins une lumière de l'ensemble de lumières ;
l'au moins une section latérale de déclenchement positionnée adjacente à un portail de fenêtre de l'ensemble de portails de fenêtre ;
un ensemble de viroles (1505, 1509, 1513, 1517) ; et,
au moins une virole, de l'ensemble de viroles, positionnée sur l'au moins une fibre optique de l'ensemble de fibres optiques.

2. Fil chirurgical selon la revendication 1 comprenant en outre :

un ensemble de réflecteurs (1550, 1551, 1552, 1553) ; et,
au moins un réflecteur, de l'ensemble de réflecteurs, adjacent à l'au moins une section latérale de déclenchement de l'ensemble de sections latérales de déclenchement.

3. Fil chirurgical selon la revendication 1 comprenant en outre :

un ensemble de chambres de diffusion ;
au moins une chambre de diffusion de l'ensemble de chambres de diffusion reliée à l'au moins un fil de l'ensemble de fils ; et,
l'au moins une section latérale de déclenchement de l'ensemble de sections latérales de déclenchement, résidant dans l'au moins une chambre de diffusion de l'ensemble de chambres de diffusion.

4. Fil chirurgical selon la revendication 3 comprenant en outre :

un ensemble de surfaces de réflexion interne ; et,
au moins une surface de réflexion interne de l'ensemble de surfaces de réflexion interne localisée au niveau d'une extrémité distale de l'au moins une chambre de diffusion de l'ensemble de chambres de diffusion.

5. Fil chirurgical selon la revendication 2, dans lequel au moins un réflecteur de l'ensemble de réflecteurs est constitué d'un matériau non métallique ou est un substrat non métallique revêtu de TiO2, ou dans lequel l'ensemble de réflecteurs est intégré dans un panneau réfléchissant, ou dans lequel un réflecteur de l'ensemble de réflecteurs comprend en outre un prisme dirigé vers un portail de fenêtre de l'ensemble de portails de fenêtre.

6. Fil chirurgical selon la revendication 1 dans lequel l'ensemble de conducteurs est agencé dans un motif radial aligné axialement.

7. Fil chirurgical selon la revendication 1 comprenant en outre :
un bouclier thermique généralement plan, adjacent à l'ensemble de fils.

8. Fil chirurgical selon la revendication 1 comprenant en outre :
un bouclier en treillis (1526) flexible généralement plan, adjacent à l'ensemble de fils.

9. Fil chirurgical selon la revendication 8, dans lequel le bouclier en treillis est un matériau en fibres de carbone, ou dans lequel le bouclier en treillis est relié à la masse sur un générateur d'impulsions implantable par un conducteur de l'ensemble de conducteurs, ou dans lequel le bouclier en treillis comprend en outre une surface réfléchissante non métallique.

10. Fil chirurgical selon la revendication 8, dans lequel le bouclier en treillis est relié à la masse sur un générateur d'impulsions implantable par un conducteur de l'ensemble de conducteurs et au moins un fil de l'ensemble de fils comprend en outre une couche conductrice non métallique, reliée à la masse sur un générateur d'impulsions implantable.

11. Fil chirurgical selon la revendication 1 dans lequel une fibre optique de l'ensemble de fibres optiques est un matériau de polyméthylméthacrylate.

12. Fil chirurgical selon la revendication 1 dans lequel l'ensemble de fils comprend en outre :

une première paire de fils pour transmission d'un premier signal de lumière ; et,
une seconde paire de fils pour réception d'un second signal de lumière, ou dans lequel l'ensemble de fils comprend en outre :

un premier fil pour transmission d'un premier signal de lumière ; et,
un second fil pour réception d'un second signal de lumière.

13. Fil chirurgical selon la revendication 1 dans lequel l'au moins une virole de l'ensemble de viroles est liée à l'au moins une fibre optique de l'ensemble de fibres optiques.

FIG. 1
(PRIOR ART)

PULSE
GENERATOR

CONTROLLER

FIG. 4
(PRIOR ART)

FIG. 2
(PRIOR ART)

FIG. 3
(PRIOR ART)

FIG. 5

FIG. 6A

501

c
z
a
x

(TOP)

## FIG. 6B

501

b
y
a
x

(FRONT)

## FIG. 6C

501

b
y
c
z

(SIDE)

## FIG. 6D

yz-PLANE

xy-PLANE

zx-PLANE

y

b

c

z

x

a

## FIG. 6E

FIG. 6F

**FIG. 7A**

EP 3 983 052 B1

FIG. 7B

FIG. 7C

EP 3 983 052 B1

**FIG. 7D**

FIG. 7E

FIG. 8

FIG. 9A

FIG. 9B

10B

1006

1008

1000

1001

10B

## FIG. 10A

1006

1001

1008

1000

## FIG. 10B

11B

1006

1101

1110
1106

1110
1106

1110
1106

1110
1106

1104

11B

## FIG. 11A

1101

1110

1106

1108

1104

1106

1110

1102

## FIG. 11B

1104

1106

1106

1106

FIG. 11C

1108

1108

1108

1106

1106

1106

1104

FIG. 11D

1204    1205

1212    814

1207    1206

1210

**FIG. 12A**

1208

814

1210

**FIG. 12B**

1212    1204

1207    1205

1210    1206

**FIG. 12C**

1219 1217 1215 1213

1211

D 1296  D 1294  E 1292  D 1290

FIG. 12D

EMITTER CURRENT — b

LIGHT OUTPUT
WITH CORRECTION — c

LIGHT OUTPUT
WITHOUT CORRECTION — a

0 — t

FIG. 12E

1260

1262 — ( START )

1264 — SET OUTPUT CURRENT ON EMITTER

1266 — READ DETECTOR VOLTAGE X

1268 — STORE X

1270 — RUN STIMULATION PROGRAM

1272 — TIMER EXPIRED? — NO

YES

1274 — READ DETECTOR VOLTAGE

IS DETECTOR VOLTAGE < X? — NO

1276

YES

1278 — INCREASE EMITTER CURRENT

NO — IS EMITTER CURRENT MAX?

1280

YES

1282 — SEND SIGNAL

FIG. 12F

1250 1251 1252

1201

1254

**FIG. 12G**

1201

1209

1254

**FIG. 12H**

FIG. 12I

FIG. 12J

**FIG. 13A**

EP 3 983 052 B1

FIG. 13B

FIG. 13C

FIG. 13D

EP 3 983 052 B1

**FIG. 13E**

FIG. 13F

EP 3 983 052 B1

**FIG. 13G**

EP 3 983 052 B1

FIG. 13H

EP 3 983 052 B1

FIG. 13J

FIG. 13I

FIG. 13K

FIG. 14A

**FIG. 14B**

1500

1546 1502 1544 1552 1542 1521 1526 14C

1583 1525 1582 1581 1519 1540

1553 1523 1551 1580

1538 1550

1530 1516 1512 1531 1532 1508 1504 1533 1535

1537 14C

1536

EP 3 983 052 B1

FIG. 14C

FIG. 15A

FIG. 15B

FIG. 16A

FIG. 16B

FIG. 16C

FIG. 17A

FIG. 17B

1900

1902 — MAKE LAMINOTOMY

1904 — PLACE ELECTRODES IN SPINAL CANAL

1906 — ANCHOR ELECTRODES

1908 — MAKE IPG POCKET

1910 — TUNNEL LEADS TO IPG POCKET

1914 — SECURE MULTI-DUCT LEADS IN IPG HEADER

1916 — PLACE IPG IN POCKET

1918 — CLOSE

**FIG. 18**

2000

2002 — INSERT TOUHY NEEDLE AND NEEDLE STYLET

2004 — REMOVE NEEDLE STYLET

2006 — INSERT PERCUTANEOUS LEAD AND STYLET

2008 — GUIDE PERCUTANEOUS LEAD TO PROPER LOCATION

2010 — REMOVE STYLET CHANNEL

2012 — INSERT OPTICAL FIBER INTO STYLET CHANNEL

2014 — REMOVE TOUHY NEEDLE

2016 — SECURE PERCUTANEOUS LEAD IN IPG HEADER

**FIG. 19**

2012

2102 — ALIGN SEMICYLINDERS
WITH LEAD BODY

↓

2104 — ASSEMBLE SEMICYLINDERS

↓

2106 — INSERT LEAD BODY INTO
THREADING ASSEMBLY

↓

2108 — INSERT FIBER INTO
THREADING ASSEMBLY

↓

2110 — THREAD FERRULE
INTO OPTICAL FIBER

↓

2112 — DISASSEMBLE
THREADING ASSEMBLY

↓

2114 — REMOVE SEMICYLINDERS

FIG. 20

**EP 3 983 052 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10035019 B, Wolf II
- US 6011993 A, Tziviskos
- US 6324428 B, Weinberg
- US 7742817 B, Malinowski
- US 20140074182 A, Wolf
- US 2018326219 A
- US 8239038 B
- US 8543213 B
- US 9132273 B
- US 9656097 B, Wolf II